# EUROPEAN PATENT APPLICATION

(11) **EP 4 736 862 A1**
(43) Date of publication of application: **06.05.2026**
(21) Application number: 24830074.1
(22) Date of filing: 12.04.2024
(51) Int. Cl.: A61K 33/10, A61K 33/06, A61K 33/08, A61K 38/26, A61K 38/22, A61K 47/18, C07C 235/52, C07C 235/60, A61P 3/10, A61P 3/04, A61P 19/10

(54) **COMPOSITION PROVIDING IMPROVED ORAL BIOAVAILABILITY OF DRUG AND METHOD THEREFOR**

(30) Priority: 30.06.2023 CN 202310790339
(71) Applicant: Hang Zhou Sciwind Biosciences Co., Ltd., Hangzhou, Zhejiang 310015 (CN); Sciwind Biosciences (Beijing) Co., Ltd., Beijing 100176 (CN)
(72) Inventor: WANG, Hongyang, Beijing 100176 (CN); JIANG, Shiyue, Beijing 100176 (CN); GUO, Wanjun, Beijing 100176 (CN); PAN, Hai, Beijing 100176 (CN)
(74) Representative: Engelhard, Markus
(86) International application number: PCT/CN2024/087583
(87) International publication number: WO 2025/001424

(57) **Abstract**

A composition providing improved oral bioavailability of a drug and a method therefor. Also disclosed is the use of a delivery synergist in the improvement of the oral bioavailability of a protein or polypeptide drug. When the delivery synergist is added to the pharmaceutical composition at a certain ratio relative to a delivery agent, the delivery effect of the delivery agent can be maximized, which can not only reduce the use amount of the delivery agent, but can also improve the oral bioavailability of the drug.

## Description

### TECHNICAL FIELD

The present application relates to the field of biomedicine, and particularly to a composition providing improved oral bioavailability of a drug and a method therefor.

### BACKGROUND

Oral administration has been the first choice of mainstream therapeutic drugs due to its simple operation, safety, effectiveness, variety of developable formulation forms, and the like. It is also favored by patients and has good patient compliance. However, many drugs with high demand are still administered by other routes, such as injection, due to various factors. For example, most polypeptide drugs such as insulin and GLP-1 agonists are currently administered by injection, which has the defect of low patient adaptability, making the administration to patients more difficult and causing long-term pain.

Oral modification of drugs that are difficult to administer orally has been one of the important directions in drug research. At present, the mainstream oral administration regimen for drugs that are difficult to administer orally (such as protein and polypeptide drugs) is to prepare oral formulations with active drugs through oral delivery agents, such as sodium caprylate (C₈Na), sodium caprate (C₁₀Na), sodium laurate (C₁₂Na), and sodium chenodeoxycholate. A relatively successful example of oral drug development is the GLP-1R agonist drug, semaglutide tablet (Rybelsus^{®}), developed by Novo Nordisk, which increases the oral absorption availability of polypeptides by a regimen of adding a delivery agent, sodium N-(8-(2-hydroxybenzoyl)amino)caprylate (SNAC), to the GLP-1R agonist tablet. This drug has been approved for marketing in the United States, Europe, and other regions. However, the greatest difficulty in the current technology of oral protein or polypeptide drugs is still the delivery efficiency of drugs, and lower delivery efficiency leads to higher raw material costs. This is mainly because the presence of the gastrointestinal barrier makes it difficult for oral protein and polypeptide drugs to be delivered to the circulatory system *in vivo.*

Therefore, there is an urgent problem to be solved in the art to improve the oral bioavailability of drugs that are difficult to administer orally (such as protein and polypeptide drugs), thereby saving the production cost of the drugs and benefiting more patients.

### CONTENT OF THE PRESENT INVENTION

In order to achieve the object described above, the present application adopts the following technical solutions.

In a first aspect, the present application provides use of a delivery synergist in improving the oral bioavailability of a protein or polypeptide drug, wherein the protein or polypeptide drug is used in combination with the delivery synergist, and the delivery synergist includes hydrotalcite, aluminum phosphate, calcium carbonate, aluminum hydroxide, sucralfate, calcium bicarbonate, aluminum bicarbonate, magnesium bicarbonate, and/or magnesium hydroxide; the protein or polypeptide drug is delivered by a delivery agent.

In some preferred technical solutions, the delivery synergist includes hydrotalcite, aluminum phosphate, calcium carbonate, aluminum hydroxide, and/or sucralfate.

In the use provided in the first aspect of the present application, the protein or polypeptide drugs may be of any type commonly used in the art, including but not limited to, a hormone, a growth factor, a cytokine, an analgesic peptide, an enzyme, a coagulation factor, a peptide neurotransmitter, and/or an antibody.

In the use provided in the first aspect of the present application, the protein or polypeptide drug may further include a receptor agonist or antagonist commonly used in the art.

In some preferred technical solutions, the receptor agonist or antagonist includes a GLP-1 receptor agonist, a GLP-1 receptor antagonist, a GIP receptor agonist, a GIP receptor antagonist, and/or an insulin receptor agonist.

Further, the receptor agonist or antagonist includes a GLP-1 receptor agonist.

Still further, the GLP-1 receptor agonist includes an exendin, GLP-1 or an analog, fragment, derivative, fusion protein, or conjugate thereof, or a pharmaceutically acceptable salt of the molecule described above.

In some of the most preferred technical solutions, the GLP-1 receptor agonist has a structure represented by formula (I):

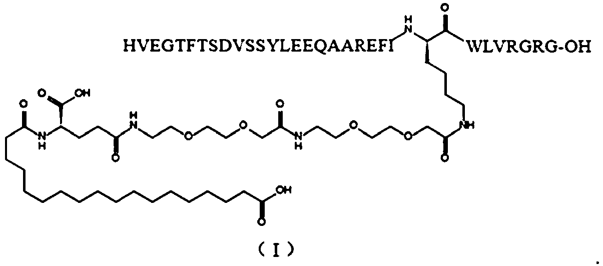

In the use provided in the first aspect of the present application, the delivery agent may have a structure represented by formula (III): 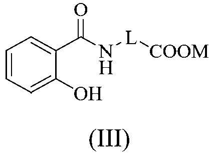 in formula (III), L represents linear or branched C5-C11 alkylene, and M represents Na⁺, K⁺, or NH₄⁺.

Further, L represents linear or branched C7-C9 alkylene, and M represents K⁺.

Still further, L represents linear C7-C9 alkylene, and M represents K⁺.

In some preferred technical solutions, the delivery agent includes a salt of N-(8-(2-hydroxybenzoyl)amino)caprylic acid.

Further, the salt of N-(8-(2-hydroxybenzoyl)amino)caprylic acid includes a sodium salt, a potassium salt, and/or an ammonium salt.

In some of the most preferred technical solutions, the salt of N-(8-(2-hydroxybenzoyl)amino)caprylic acid is selected from a potassium salt, which has a structure represented by formula (II):

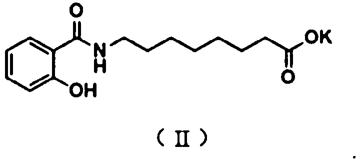

In the use provided in the first aspect of the present application, the delivery agent may also have a structure represented by formula (IV):

CH₃-(CH₂)n-COOM, (IV)

in formula (IV), n represents an integer from 4 to 10, and M represents Na⁺, K⁺, or NH₄⁺. Further, n represents an integer from 6 to 8, and M represents Na⁺.

In some preferred technical solutions, the delivery agent includes a salt of caprylic acid, capric acid, and/or lauric acid.

Further, the delivery agent includes a sodium salt, a potassium salt, and/or an ammonium salt of caprylic acid, capric acid, and/or lauric acid.

In some of the most preferred technical solutions, the delivery agent includes a sodium salt of caprylic acid, capric acid, and/or lauric acid.

In the use provided in the first aspect of the present application, the delivery agent may further include a salt of chenodeoxycholic acid.

Further, the delivery agent includes a sodium salt, a potassium salt, and/or an ammonium salt of chenodeoxycholic acid.

In some preferred technical solutions, the delivery agent includes sodium chenodeoxycholate.

In the use provided in the first aspect of the present application, the delivery agent and the delivery synergist are in a mass ratio of 1:0.3-1:3.

In some preferred technical solutions, the delivery agent and the delivery synergist are in a mass ratio of 1:0.3-1:2.

In the use provided in the first aspect of the present application, the combination use comprises simultaneously using the protein or polypeptide drug and the delivery synergist in the form of a pharmaceutical composition; or separately using the protein or polypeptide drug and the delivery synergist in a specific order, for example, using the delivery synergist first and then using the protein or polypeptide drug.

Further, the combination use is to simultaneously use the protein or polypeptide drug and the delivery synergist in the form of a pharmaceutical composition.

In the use provided in the first aspect of the present application, the protein or polypeptide drug and the delivery agent are in a mass ratio of 1:5-1:150.

In some preferred technical solutions, the protein or polypeptide drug and the delivery agent are in a mass ratio of 1:10-1:50; for example, the mass ratio may be 1:10-1:30.

In the use provided in the first aspect of the present application, the pharmaceutical composition further comprises a pharmaceutically acceptable carrier, which may be of any type commonly used in the art, including but not limited to one or more of a lubricant, a filler, a disintegrant, a flavoring agent, a colorant, a binder, an forming agent , a buffer, a diluent, a solubilizer, a tonicity regulator, a surfactant, a preservative, an isotonic agent, a stabilizer, and a chelating agent.

In some preferred technical solutions, the pharmaceutically acceptable carrier includes a lubricant, a filler, and a disintegrant.

Further, the lubricant includes magnesium stearate, glyceryl tribehenate, and/or stearic acid.

In some of the most preferred technical solutions, the lubricant is selected from magnesium stearate; and/or the filler is selected from mannitol and/or microcrystalline cellulose; and/or the disintegrant is selected from croscarmellose sodium.

In the use provided in the first aspect of the present application, the pharmaceutical composition further comprises one or more active pharmaceutical ingredients for use in combination with the protein or polypeptide drug.

In some preferred technical solutions, the active pharmaceutical ingredient is selected from an antidiabetic agent, an anti-obesity agent, an appetite-regulating agent, an antihypertensive agent, an agent for treating and/or preventing complications and conditions caused by or associated with diabetes, and an agent for treating and/or preventing complications and conditions caused by or associated with obesity.

In the use provided in the first aspect of the present application, a dosage form of the pharmaceutical composition may be any dosage form commonly used in the art.

In some preferred technical solutions, the dosage form of the pharmaceutical composition may be an oral dosage form, including but not limited to, one or more of a tablet, a capsule, an emulsion, an aqueous suspension, a dispersant, and a powder.

In some of the most preferred technical solutions, the dosage form of the pharmaceutical composition is a tablet.

In some of the most preferred technical solutions, the tablet may be prepared by a dry granulation process and/or a direct mixing process commonly used in the art.

In some preferred technical solutions, the tablet has a hardness of 30-90 N.

In a second aspect, the present application provides a method for improving the oral bioavailability of a protein or polypeptide drug, which comprises: using the protein or polypeptide drug in combination with a delivery synergist, wherein the delivery synergist includes hydrotalcite, aluminum phosphate, calcium carbonate, aluminum hydroxide, sucralfate, calcium bicarbonate, aluminum bicarbonate, magnesium bicarbonate, and/or magnesium hydroxide; the protein or polypeptide drug is delivered by a delivery agent.

In some preferred technical solutions, the delivery synergist includes hydrotalcite, aluminum phosphate, calcium carbonate, aluminum hydroxide, and/or sucralfate.

In the method provided in the second aspect of the present application, the protein or polypeptide drug is as defined in the first aspect of the present application.

In the method provided in the second aspect of the present application, the delivery agent is as defined in the first aspect of the present application.

In the method provided in the second aspect of the present application, the mode of the combination use is as defined in the first aspect of the present application.

In the method provided in the second aspect of the present application, both the mass ratio of the delivery agent to the delivery synergist and the mass ratio of the protein or polypeptide drug to the delivery agent are as defined in the first aspect of the present application.

In the method provided in the second aspect of the present application, the pharmaceutical composition further comprises a pharmaceutically acceptable carrier as defined in the first aspect of the present application.

In the method provided in the second aspect of the present application, the pharmaceutical composition further comprises one or more active pharmaceutical ingredients for use in combination with the protein or polypeptide drug, and the active pharmaceutical ingredient is as defined in the first aspect of the present application.

In the method provided in the second aspect of the present application, the dosage form of the pharmaceutical composition and the preparation method therefor may be any dosage form and process commonly used in the art, for example, as defined in the first aspect of the present application.

In a third aspect, the present application provides a pharmaceutical composition having improved oral bioavailability and having a protein or polypeptide drug as an active ingredient, wherein the pharmaceutical composition comprises a delivery synergist, and the delivery synergist includes hydrotalcite, aluminum phosphate, calcium carbonate, aluminum hydroxide, sucralfate, calcium bicarbonate, aluminum bicarbonate, magnesium bicarbonate, and/or magnesium hydroxide; the pharmaceutical composition further comprises a delivery agent.

In some preferred technical solutions, the delivery synergist includes hydrotalcite, aluminum phosphate, calcium carbonate, aluminum hydroxide, and/or sucralfate.

In the pharmaceutical composition provided in the third aspect of the present application, the protein or polypeptide drug is as defined in the first aspect of the present application.

In the pharmaceutical composition provided in the third aspect of the present application, the delivery agent is as defined in the first aspect of the present application.

In the pharmaceutical composition provided in the third aspect of the present application, both the mass ratio of the delivery agent to the delivery synergist and the mass ratio of the protein or polypeptide drug to the delivery agent are as defined in the first aspect of the present application. In the pharmaceutical composition provided in the third aspect of the present application, the protein or polypeptide drug is used in combination with the delivery synergist, and the mode of combination use is as defined in the first aspect of the present application.

In the pharmaceutical composition provided in the third aspect of the present application, the pharmaceutical composition further comprises a pharmaceutically acceptable carrier as defined in the first aspect of the present application.

In the pharmaceutical composition provided in the third aspect of the present application, the pharmaceutical composition further comprises one or more active pharmaceutical ingredients for use in combination with the protein or polypeptide drug, and the active pharmaceutical ingredient is as defined in the first aspect of the present application.

In the pharmaceutical composition provided in the third aspect of the present application, the dosage form of the pharmaceutical composition and the preparation method therefor may be any dosage form and process commonly used in the art, for example, as defined in the first aspect of the present application.

In a fourth aspect, the present application provides use of a substance shown below as a synergist for a drug delivery agent: hydrotalcite, aluminum phosphate, calcium carbonate, aluminum hydroxide, sucralfate, calcium bicarbonate, aluminum bicarbonate, magnesium bicarbonate, and/or magnesium hydroxide, wherein the delivery agent is as defined in the first aspect of the present application.

In a fifth aspect, the present application provides a pharmaceutical composition comprising a delivery agent and a delivery synergist, wherein the delivery agent is as defined in the first aspect of the present application, and the delivery synergist includes hydrotalcite, aluminum phosphate, calcium carbonate, aluminum hydroxide, sucralfate, calcium bicarbonate, aluminum bicarbonate, magnesium bicarbonate, and/or magnesium hydroxide.

In some preferred technical solutions, the delivery synergist includes hydrotalcite, aluminum phosphate, calcium carbonate, aluminum hydroxide, and/or sucralfate.

In the pharmaceutical composition provided in the fifth aspect of the present application, the pharmaceutical composition further comprises a protein or polypeptide drug as defined in the first aspect of the present application.

In the pharmaceutical composition provided in the fifth aspect of the present application, both the mass ratio of the delivery agent to the delivery synergist and the mass ratio of the protein or polypeptide drug to the delivery agent are as defined in the first aspect of the present application.

In the pharmaceutical composition provided in the fifth aspect of the present application, the protein or polypeptide drug is used in combination with the delivery synergist, and the mode of combination use is as defined in the first aspect of the present application.

In the pharmaceutical composition provided in the fifth aspect of the present application, the pharmaceutical composition further comprises a pharmaceutically acceptable carrier as defined in the first aspect of the present application.

In the pharmaceutical composition provided in the fifth aspect of the present application, the pharmaceutical composition further comprises one or more active pharmaceutical ingredients for use in combination with the protein or polypeptide drug, and the active pharmaceutical ingredient is as defined in the first aspect of the present application.

In the pharmaceutical composition provided in the fifth aspect of the present application, the dosage form of the pharmaceutical composition and the preparation method therefor may be any dosage form and process commonly used in the art, for example, as defined in the first aspect of the present application.

In a sixth aspect, the present application provides use of the pharmaceutical composition according to any one of the third aspect of the present application and the fifth aspect of the present application as a medicament (i.e., for therapeutic use).

In a seventh aspect, the present application provides use of the pharmaceutical composition according to any one of the third aspect of the present application and the fifth aspect of the present application in the manufacture of a medicament for preventing and/or treating a metabolism-related disease, a nerve-related disease, or a brain-related disease.

In an eighth aspect, the present application provides a method for preventing and/or treating a metabolism-related disease, a nerve-related disease, or a brain-related disease, which comprises: administering to a patient in need thereof a therapeutically effective dose of the pharmaceutical composition according to any one of the third aspect of the present application and the fifth aspect of the present application.

In the seventh aspect and the eighth aspect of the present application, the metabolism-related disease includes a carbohydrate metabolism disorder and/or a lipid metabolism disorder.

In some preferred technical solutions, the carbohydrate metabolism disorder includes a disease related to abnormal blood glucose.

Further, the disease related to abnormal blood glucose includes diabetes, diabetic oculopathy, diabetic heart disease, diabetic nephropathy, diabetic neuropathy, necrosis of distal lower limbs, atherosclerosis, coronary heart disease, myocardial infarction, cerebral thrombosis, cerebral hemorrhage, cerebral embolism, osteoporosis, hyperlipidemia, hypertension, obesity, fatty liver, and/or cirrhosis.

Still further, the diabetes includes hyperglycemia, type II diabetes, impaired glucose tolerance, type I diabetes, non-insulin-dependent diabetes, maturity-onset diabetes of the young (MODY), and/or gestational diabetes.

In some preferred technical solutions, the lipid metabolism disorder includes a disease related to abnormal body weight.

Further, the disease related to abnormal body weight includes anemia, gastroptosis, inflammatory bowel disease, dyspepsia, gastrointestinal ulcer, endocrine dyscrasia, osteoporosis, obesity, hypertension, hyperlipidemia, coronary heart disease, diabetes, fatty liver, joint deformity, pain, cancer, respiratory insufficiency, kidney diseases, hyperlipidemia, atherosclerosis, cirrhosis, angina pectoris, and/or myocardial infarction.

In the seventh aspect and the eighth aspect of the present application, the nerve-related disease includes a neurodegenerative disease

Further, the neurodegenerative disease includes brain atrophy, cerebral ischemia, brain injury, epilepsy, Parkinson's disease, Alzheimer's disease, amyotrophic lateral sclerosis, multiple sclerosis, Huntington's disease, spinocerebellar ataxia, Pick's disease, bovine spongiform encephalopathy, and/or Creutzfeldt-Jakob disease.

In a ninth aspect, the present application provides a kit comprising the pharmaceutical composition according to any one of the third aspect of the present application and the fifth aspect of the present application.

The present application has the following advantages and beneficial effects:
After the delivery synergist described in the present application is added to the pharmaceutical composition provided in the present application at a certain ratio of the delivery synergist to a delivery agent, the delivery effect of the delivery agent can be maximized, so that the amount of the delivery agent used can be reduced, and the oral bioavailability of a drug (e.g., a protein or polypeptide drug) can be increased.

### BRIEF DESCRIPTION OF THE DRAWINGS

FIG. 1 shows oral absorption curves of the GLP-1 receptor agonist after calcium carbonate and aluminum hydroxide were separately added to the composition in Example 1.
FIG. 2 shows plasma concentration-time curves for the compositions in a pharmacokinetic study in cynomolgus monkeys on day 7 in Example 3.

### DETAILED DESCRIPTION OF THE PREFERRED EMBODIMENTS

Definitions of some terms used in this specification are provided below. Unless otherwise stated, all technical and scientific terms used herein generally have the same meaning as commonly understood by those of ordinary skill in the art to which the present application pertains.

In the present application, "protein or polypeptide drug" refers to a protein or polypeptide compound contained in an oral drug, which functions directly as an active pharmaceutical ingredient or indirectly assists an active pharmaceutical ingredient in preventing and/or treating diseases *in vivo.*

In some embodiments of the present application, the protein or polypeptide drug may be a natural protein or polypeptide compound (e.g., a hormone, a growth factor, a cytokine, an analgesic peptide, an enzyme, a coagulation factor, a peptide neurotransmitter, and an antibody) or an artificially synthesized polypeptide or protein, as well as an analog, a derivative, a conjugate, or a fusion protein of the polypeptide or protein described above, or a pharmaceutically acceptable salt thereof.

In some embodiments of the present application, the protein or polypeptide drug may be a compound having the active function of a receptor agonist or antagonist. The receptor agonist or antagonist includes, but is not limited to: a GLP-1 receptor agonist, a GLP-1 receptor antagonist, a GIP receptor agonist, a GIP receptor antagonist, an Amylin receptor agonist, a heparin receptor agonist, a growth hormone receptor agonist, an interferon receptor agonist, an interleukin receptor agonist, a follicle-stimulating hormone receptor agonist, a gonadotropin receptor agonist, an erythropoietin receptor agonist, a PYY (peptide YY) receptor agonist, an oxyntomodulin receptor agonist, a GLP-2 (glucagon-like peptide-2) receptor agonist, a calcitonin receptor agonist, a PTH (parathyroid hormone) receptor agonist, a ghrelin receptor agonist, an endocannabinoid receptor agonist, a leptin receptor agonist, a serotonin receptor agonist, a fibroblast growth factor 21 (FGF21) receptor agonist, a cholecystokinin (CCK) receptor agonist, and a glucagon receptor agonist.

In some embodiments of the present application, the protein or polypeptide drug comprises the active functions of one or more receptor agonists or antagonists.

In some embodiments of the present application, the protein or polypeptide drug is preferably a GLP-1 receptor agonist; the GLP-1 receptor agonist includes compounds with GLP-1 receptor agonistic activity, particularly polypeptides/proteins, and analogs, derivatives, conjugates, or fusion proteins of the polypeptides/proteins described above, or pharmaceutically acceptable salts thereof.

In some embodiments of the present application, the GLP-1 receptor agonist includes an exendin, an exendin analog, an exendin agonist, GLP-1 (7-37), a GLP-1 (7-37) analog, a derivative, conjugate, or fusion protein thereof, or a pharmaceutically acceptable salt thereof. The GLP-1 receptor agonist compound may optionally be amidated. The terms "GLP-1 receptor agonist" and "GLP-1 receptor agonist compound" have the same meaning.

Among them, the exendin includes a naturally occurring exendin (or a synthetic version of a naturally occurring one), which is found in the salivary secretion of *Heloderma suspectum.* The exendin of particular interest includes exendin-3 and exendin-4. The exendin, exendin analog, and exendin agonist used in the present application may optionally be amidated and may also exist in an acid form, a pharmaceutically acceptable salt form, or any other physiologically active form of the molecule.

The GLP-1 (7-37) analog refers to a peptide that can elicit bioactivity similar to that of GLP-1 (7-37) when assessed by measurement methods known in the art, such as the receptor binding assay or *in vivo* blood glucose assay, e.g., as described by Hargrove et al., Regulatory Peptides, 141:113-119 (2007), the disclosure of which is incorporated herein by reference. In one embodiment, the term "GLP-1 (7-37) analog" refers to a peptide having an amino acid sequence with 1, 2, 3, 4, 5, 6, 7, or 8 amino acid substitutions, insertions, deletions, or a combination of two or more thereof when compared to the amino acid sequence of GLP-1 (7-37). The GLP-1 (7-37) analog includes the amidated form, acid form, and any other physiologically active form of the molecule.

In the present application, "agonist" refers to a compound, molecule, or agent capable of stimulating bioactivity.

In the present application, "analog" refers to a substance having a similar structure and/or function to the protein or polypeptide of the present application.

In the present application, "pharmaceutically acceptable salt" refers to any adduct between two or more chemical substances capable of undergoing proton transfer. Thus, pharmaceutically acceptable salts include adducts in which complete proton transfer has occurred; adducts in which partial proton transfer has occurred (e.g., in which an equilibrium mixture of charged and uncharged substances is formed); and/or adducts in which proton transfer has not occurred but the chemical substances are bound, for example, by hydrogen bonds. It should be understood that pharmaceutically acceptable salts also encompass adducts in which a tight ion pair is present. It should also be understood that pharmaceutically acceptable salts encompass a continuum of adducts between those adducts in which complete proton transfer has occurred to form discrete ions and/or adducts in which two substances are bound but proton transfer has not occurred or has only partially occurred. See, e.g., Childs et al., Mol. Pharmaceutics, 2007, 4(3), pp 323-338. A given pharmaceutically acceptable salt can contain one or more adducts on this continuum.

Pharmaceutically acceptable salts include salts with acidic or basic groups. Pharmaceutically acceptable acid addition salts include, but are not limited to, hydrochloride, hydrobromide, hydroiodide, nitrate, methanesulfonate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate, and pamoate (i.e., 1,1'-methylene-bis-(2-hydroxy-3-naphthoate)). Suitable basic salts include, but are not limited to, aluminum, calcium, lithium, magnesium, potassium, sodium, zinc, and diethanolamine salts.

In the present application, the mass ratio of the delivery agent to the delivery synergist may be any mass ratio capable of improving the delivery effect; for example, the mass ratio may be 1:0.3-1:3, including mass ratios of about 1:0.3, about 1:0.4, about 1:0.5, about 1:0.6, about 1:0.7, about 1:0.8, about 1:0.9, about 1:1, about 1:1.1, about 1:1.2, about 1:1.3, about 1:1.4, about 1:1.5, about 1:1.6, about 1:1.7, about 1:1.8, about 1:1.9, about 1:2, about 1:2.1, about 1:2.2, about 1:2.3, about 1:2.4, about 1:2.5, about 1:2.6, about 1:2.7, about 1:2.8, about 1:2.9, and about 1:3, as well as any mass ratio range.

In the present application, the mass ratio of the protein or polypeptide drug to the delivery agent may be any mass ratio capable of achieving delivery of the protein or polypeptide drug; for example, the mass ratio may be 1:5-1:150, including mass ratios of about 1:5, about 1:10, about 1:15, about 1:20, about 1:25, about 1:30, about 1:35, about 1:40, about 1:45, about 1:50, about 1:55, about 1:60, about 1:65, about 1:70, about 1:75, about 1:80, about 1:85, about 1:90, about 1:95, about 1:100, about 1:105, about 1:110, about 1:115, about 1:120, about 1:125, about 1:130, about 1:135, about 1:140, about 1:145, and about 1:150, as well as any mass ratio range.

In the present application, "bioavailability" refers to the extent and rate at which a substance (e.g., a drug, peptide, or hormone) is available at a physiologically active site. In the field of pharmacology, the term "bioavailability" is understood as the fraction of an administered dose of an unchanged drug, compound, or medicament that reaches the systemic circulation, and is one of the main pharmacokinetic properties of a drug. By definition, when a drug is administered intravenously, its bioavailability is 100%. However, when a drug is administered by other routes (e.g., oral administration or intramuscular administration), its bioavailability is usually reduced due to factors such as incomplete absorption and first pass metabolism. These factors may vary from patient to patient due to, e.g., differences in individual metabolism.

Standard tests for determining the bioavailability of a drug are known to those skilled in the art, and include, in particular, measuring the relative area under the curve (AUC) of the concentration of the drug administered orally and intravenously (i.v.) in the same species.

In the present application, the salt of N-(8-(2-hydroxybenzoyl)amino)caprylic acid may be crystalline and/or amorphous, including but not limited to, sodium salt, potassium salt, and/or ammonium salt.

In the present application, "pharmaceutically acceptable carrier" refers to a non-toxic material that does not interfere with the effect of the active pharmaceutical ingredient or pharmaceutical composition of the present application or the bioactivity of the active pharmaceutical ingredient or pharmaceutical composition of the present application. It is known in the art to prepare active pharmaceutical ingredients with pharmaceutically acceptable carriers, e.g., Remington: The Science and Practice of Pharmacy (e.g., 21st edition (2005), and any later editions). Non-limiting examples of the pharmaceutically acceptable carrier include, but are not limited to, salts (e.g., acidic/anionic salts and basic/cationic salts), forming agents, buffers, diluents, solubilizers, tonicity regulators, surfactants, preservatives, isotonic agents, flavoring agents, colorants, stabilizers, binders, disintegrants, fillers, lubricants, and chelating agents. One or more pharmaceutically acceptable carriers may be used to prepare the pharmaceutical composition of the present application.

In some embodiments of the present application, the pharmaceutically acceptable carrier includes an acidic/anionic salt. Non-limiting examples of the acidic salt/anionic salt include, but are not limited to, acetate, benzenesulfonate, benzoate, bicarbonate, bitartrate, bromide, calcium edetate, camphorsulfonate, carbonate, chloride, citrate, dihydrochloride, edetate, edisylate, estolate, ethanesulfonate, fumarate, glucoheptonate, gluconate, glutamate, glycollyl arsanilate, hexylresorcinate, hydrabamine, hydrobromide, hydrochloride, hydroxynaphthoate, iodide, isethionate, lactate, lactobionate, malate, maleate, mandelate, methanesulfonate, methylbromide, methylnitrate, methylsulfate, mucate, naphthalenesulfonate, nitrate, pamoate, pantothenate, phosphate/diphosphate, polygalacturonate, salicylate, stearate, subacetate, succinate, sulfate, tannate, tartrate, teoclate, tosylate, triethiodide, and combinations thereof.

In some embodiments of the present application, the pharmaceutically acceptable carrier includes a basic/cationic salt. Non-limiting examples of the basic/cationic salt include, but are not limited to, aluminum, 2-amino-2-hydroxymethyl-propane-1,3-diol (also known as tris(hydroxymethyl)aminomethane, tromethamine, or TRIS), ammonia, benzathine benzylpenicillin, tert-butylamine, chloroprocaine, choline, cyclohexylamine, diethanolamine, ethylenediamine, lithium, L-lysine, magnesium, meglumine, N-methyl-D-glucamine, piperidine, potassium, procaine, quinine, sodium, triethanolamine, zinc, and combinations thereof.

In some embodiments of the present application, the pharmaceutically acceptable carrier includes a buffer. Non-limiting examples of the buffer include, but are not limited to, arginine, aspartic acid, dihydroxyethylglycine, citrate, disodium hydrogen phosphate, fumaric acid, glycine, glycylglycine, histidine, lysine, maleic acid, malic acid, sodium acetate, sodium carbonate, sodium dihydrogen phosphate, sodium phosphate, succinate, tartaric acid, triazine, tris(hydroxymethyl)aminomethane, and combinations thereof.

In some embodiments of the present application, the pharmaceutically acceptable carrier includes a preservative. Non-limiting examples of the preservative include, but are not limited to, benzethonium chloride, benzoic acid, benzyl alcohol, bromonitropropanediol, butyl 4-hydroxybenzoate, chlorobutanol, chlorocresol, chlorhexidine, chlorphenesin, o-cresol, m-cresol, p-cresol, ethyl 4-hydroxybenzoate, imidurea, methyl 4-hydroxybenzoate, phenol, 2-phenoxyethanol, 2-phenylethanol, propyl 4-hydroxybenzoate, sodium dehydroacetate, thiomersal, and combinations thereof.

In some embodiments of the present application, the pharmaceutically acceptable carrier includes an isotonic agent. Non-limiting examples of the isotonic agent include, but are not limited to, amino acids (e.g., glycine, histidine, arginine, lysine, isoleucine, aspartic acid, tryptophan, and threonine), sugar alcohols (e.g., glycerol, 1,2-propanediol, propylene glycol, 1,3-propanediol, and 1,3-butanediol), polyethylene glycol (e.g., PEG400), and combinations thereof. Another example of the isotonic agent includes a saccharide. Non-limiting examples of the saccharide may be monosaccharides, disaccharides, polysaccharides, or water-soluble glucans, including, for example, fructose, glucose, mannose, sorbose, xylose, maltose, lactose, sucrose, trehalose, dextran, amylopectin, dextrin, cyclodextrin, α- and β-HPCD, soluble starch, hydroxyethyl starch, sodium carboxymethyl cellulose, and combinations thereof. Another example of the isotonic agent is a sugar alcohol, wherein the term "sugar alcohol" is defined as a C(4-8) hydrocarbon having at least one -OH group. Non-limiting examples of the sugar alcohol include, but are not limited to, mannitol, sorbitol, inositol, galactitol, hexitol, xylitol, arabitol, and combinations thereof. Drugs containing each of the isotonic agents listed in this paragraph constitute alternative embodiments of the present application.

In some embodiments of the present application, the pharmaceutically acceptable carrier includes a chelating agent. Non-limiting examples of the chelating agent include, but are not limited to, salts of citric acid, aspartic acid, and ethylenediaminetetraacetic acid (EDTA), and combinations thereof.

In some embodiments of the present application, the pharmaceutically acceptable carrier includes a stabilizer. Non-limiting examples of the stabilizer include carboxy-/hydroxycellulose and derivatives thereof (such as HPC, HPC-SL, HPC-L, and HPMC), cyclodextrin, 2-methylthioethanol, polyethylene glycol (such as PEG 3350), polyvinyl alcohol (PVA), polyvinylpyrrolidone, salts (such as sodium chloride), sulfur-containing substances (such as thioglycerol or thioglycolic acid), and combinations thereof.

In some embodiments of the present application, the pharmaceutically acceptable carrier includes a lubricant (also referred to as a "glidant"). The lubricant may be used to prevent dosage forms from adhering to the surfaces of rollers, dies, and punches, and to reduce friction between particles. The lubricant may also facilitate ejection of dosage forms from die cavities and improve the granulation flow rate during processing. Non-limiting examples of the lubricant include, but are not limited to, magnesium stearate, glyceryl behenate, calcium stearate, zinc stearate, stearic acid, silica, talc, polyethylene glycol, mineral oil, carnauba wax, palmitic acid, sodium stearyl fumarate, sodium dodecyl sulfate, glyceryl palmitostearate, myristic acid, hydrogenated vegetable oil, fat, white petrolatum, white beeswax, sodium benzoate, sodium hyaluronate, poloxamer, glyceryl monostearate, dimethicone, mixed fatty acid glycerides (stearin), polyvinyl alcohol, leucine, light liquid paraffin, hydrogenated soybean oil, sorbitan trioleate (Span 85), glyceryl behenate, cetostearyl alcohol, octyldodecanol, liquid paraffin, stearic acid, magnesium lauryl sulfate, almond oil, rapeseed oil-low erucic acid, cetyl esters wax, glyceryl palmitostearate, isopropyl palmitate, light liquid paraffin, sodium stearyl fumarate, glyceryl behenate, potassium benzoate, sterilized corn starch, myristic acid, glyceryl tricaprylate, lauric acid, magnesium oxide, calcium phosphate, powdered cellulose, magnesium silicate, magnesium trisilicate, calcium phosphate, hydrophobic silica gel, and other known lubricants, and/or a mixture of two or more thereof. In one embodiment, the lubricant for raw material granulation, if present, is magnesium stearate.

In some embodiments of the present application, the pharmaceutically acceptable carrier includes one or more surfactants, preferably one surfactant, at least one surfactant, or two different surfactants. The surfactant refers to any molecule or ion consisting of a water-soluble portion (hydrophilic) and a lipid-soluble portion (lipophilic). For example, the surfactant is selected from: an anionic surfactant, a cationic surfactant, a nonionic surfactant, and/or a zwitterionic surfactant, and combinations thereof.

In some embodiments of the present application, the pharmaceutically acceptable carrier includes a flavoring agent. The flavoring agent may be used to improve the palatability of pharmaceutical compositions. Non-limiting examples of the flavoring agent include, but are not limited to, natural and/or synthetic (i.e., artificial) compounds having the corresponding taste (e.g., mint, spearmint, wintergreen, cinnamon, menthol, cherry, strawberry, watermelon, grape, banana, peach, pineapple, apricot, pear, raspberry, lemon, grapefruit, orange, prune, apple, fruit punch, *Passiflora quadrangularis,* chocolate (e.g., white chocolate, milk chocolate, and dark chocolate), vanilla, caramel, coffee, and hazelnut), and combinations thereof.

In some embodiments of the present application, the pharmaceutically acceptable carrier includes a colorant. The colorant may be used to stain and code a pharmaceutical composition, e.g., to indicate the type and dose of a therapeutic agent therein. Non-limiting examples of the colorant include, but are not limited to, natural and/or artificial compounds such as FD&C colorants and natural fruit juice concentrates; dyes such as titanium dioxide, silicon dioxide, and zinc oxide; and combinations thereof.

In some embodiments of the present application, the pharmaceutically acceptable carrier includes a disintegrant. Non-limiting examples of the disintegrant include, but are not limited to, sodium carboxymethyl starch, low-substituted hydroxypropyl cellulose, cross-linked polyvinylpyrrolidone , croscarmellose sodium, low-substituted hydroxypropyl methylcellulose, starch, guar gum, magnesium aluminum silicate, alginic acid, sodium alginate, methylcellulose, crospovidone, polacrilin potassium, microcrystalline cellulose, anhydrous citric acid, pregelatinized starch, calcium carboxymethylcellulose, powdered cellulose, sodium carboxymethyl starch, chitosan, calcium alginate, and combinations thereof.

In some embodiments of the present application, the pharmaceutically acceptable carrier includes a filler. Non-limiting examples of the filler include, but are not limited to, lactose, microcrystalline cellulose, starch, pregelatinized starch, mannitol, kaolin, cellulose acetate, fructose, dextrin, talc, calcium sulfate, sodium chloride, xylitol, glucose, sorbitol, anhydrous lactose, magnesium oxide, zinc oxide, ethylcellulose, sucrose, anhydrous lactose, dextran, and combinations thereof.

In some embodiments of the present application, the pharmaceutically acceptable carrier includes a binder. Non-limiting examples of the binder include, but are not limited to, low-substituted hydroxypropyl methylcellulose, starch, hydrogenated soybean oil, hydroxyethyl methylcellulose, hydroxypropyl methylcellulose, α-lactose, dextran, hydroxypropyl starch, vitamin E polyethylene glycol succinate, gum arabic, alginic acid, methylcellulose, polyacrylic resin, povidone, maltodextrin, ethylhydroxycellulose, zein, pregelatinized starch, hydroxypropyl methylcellulose, hydroxypropyl methylcellulose, carob gum, carbomer, povidone, glyceryl behenate, chitosan, liquid glucose, hydroxyethylcellulose, sunflower oil, hydrogenated vegetable oil, inulin, and combinations thereof.

In the present application, the pharmaceutical composition for oral administration may be any orally acceptable dosage form, including but not limited to capsules, tablets, powders, emulsions and aqueous suspensions, dispersants, and solutions. In the case of tablets for oral use, carriers that are commonly used include lactose and corn starch. A lubricant, such as magnesium stearate, is also generally added. For oral administration in the form of capsules, useful diluents include lactose and dry corn starch. When aqueous suspensions or emulsions are administered orally, the active ingredient may be suspended or dissolved in an oil phase combined with an emulsifier or a suspending agent. If desired, certain sweetening agents, flavoring agents, or colorants may be added. A nasal aerosol or inhalation composition may be prepared according to techniques well known in the field of pharmaceutical formulations and may be prepared as a saline solution using benzyl alcohol or other suitable preservatives, absorption promoters, fluorocarbons, and/or other solubilizing agents or dispersants known in the art. The pharmaceutical composition of the present application may also be administered rectally in the form of a suppository.

If desired, tablets may be coated by standard aqueous or non-aqueous techniques. Any pharmaceutical method may be used to prepare such dosage forms. In general, pharmaceutical compositions and dosage forms are prepared by uniformly and intimately mixing active ingredients with liquid carriers, finely divided solid carriers, or both, and then, if necessary, shaping the products into the desired forms.

In a specific embodiment of the present application, the dosage form of the pharmaceutical composition is a tablet.

In some embodiments of the present application, the preparation method for the tablet may be any conventional tablet preparation method familiar to those skilled in the art. For example, a dry granulation process may be used for preparation, or a direct mixing process may also be used for preparation.

The specific procedures of the dry granulation process are as follows: 1. Material pretreatment: The protein or polypeptide drug is sieved with a 50- to 200-mesh sieve, preferably a 100-mesh sieve; if necessary, the protein or polypeptide drug is milled first and then sieved. The delivery agent is sieved with a 20- to 100-mesh sieve, preferably a 50-mesh sieve, and the agent that does not pass through the sieve is sieved again after grinding. The delivery synergist is treated by the same method as that of the delivery agent. The lubricant is taken directly. Other pharmaceutically acceptable carriers, if present, are all sieved with a 20- to 80-mesh sieve, preferably a 40-mesh sieve. 2. First mixing: A certain amount of the protein or polypeptide drug, the delivery agent, the delivery synergist, part of the lubricant, and optionally other pharmaceutically acceptable carriers are manually mixed well to obtain a mixed powder I. 3. Dry granulation: The mixed powder I described above is compacted into strip-shaped flakes with a suitable hardness and then milled into granules with a suitable particle size. 4. Re-mixing: The granules described above are collected, the remaining lubricant is added again, and the mixture is manually mixed well. 5. Tableting: Tableting is performed according to a conventional tableting process in the art, with the hardness of the tablet controlled to be in the range of 30-90 N.

The specific procedures of the direct mixing process are as follows: 1. Material pretreatment: The protein or polypeptide drug is sieved with a 100-mesh sieve; if necessary, the protein or polypeptide drug is milled first and then sieved. The delivery agent is sieved with a 50-mesh sieve, and the agent that does not pass through the sieve is sieved again after grinding. The delivery synergist is sieved with a 20- to 80-mesh sieve, preferably a 40-mesh sieve. The lubricant is taken directly. Other pharmaceutically acceptable carriers, if present, are all sieved with a 20- to 80-mesh sieve, preferably a 40-mesh sieve. 2. Mixing: A certain amount of the protein or polypeptide drug, the delivery agent, the delivery synergist, the lubricant, and optionally other pharmaceutically acceptable carriers are manually mixed well to obtain a mixed powder I. 3. Tableting: Tableting is performed according to a conventional tableting process in the art, with the hardness of the tablet controlled to be in the range of 30-90 N.

In a specific embodiment of the present application, the tablet is prepared using a direct mixing process.

In a specific embodiment of the present application, the tablet is prepared using a granulation process, preferably dry granulation.

In the present application, in addition to the pharmaceutical composition, the kit may comprise one or more containers selected from bottles, vials, ampoules, blister packs, and syringes. The kit may further comprise one or more instructions for use in treating and/or preventing a disease, condition, or dysregulation, one or more syringes, one or more applicators, or a sterile solution suitable for reconstituting the pharmaceutical composition of the present application.

In the present application, the active pharmaceutical ingredient for use in combination may be any ingredient that is different in type from the aforementioned protein or polypeptide drugs but has the same or similar active function, or that acts together with the aforementioned protein or polypeptide drugs to produce an active function; for example, it may be selected from an antidiabetic agent, an anti-obesity agent, an appetite-regulating agent, an antihypertensive agent, an agent for treating and/or preventing complications and conditions caused by or associated with diabetes, and an agent for treating and/or preventing complications and conditions caused by or associated with obesity. Non-limiting examples of these pharmacologically active pharmaceutical ingredients include, but are not limited to, insulin, sulfonylureas, biguanides, meglitinides, glucosidase inhibitors, glucagon antagonists, DPP-IV (dipeptidyl peptidase-IV) inhibitors, and sodium-glucose transporter 2 (SGLT2) inhibitors; canagliflozin, dapagliflozin, empagliflozin, ertugliflozin, ipragliflozin, tofogliflozin, luseogliflozin, bexagliflozin, remogliflozin etabonate, and sotaglifkozin, in particular dapagliflozin and empagliflozin, inhibitors of liver enzymes involved in the stimulation of gluconeogenesis and/or glycogenolysis, glucose uptake modulators, compounds that alter lipid metabolism such as antihyperlipidemic agents, e.g., HMG-CoA inhibitors (statins), gastric inhibitory polypeptides (GIP analogs), compounds that reduce food intake, RXR agonists, and agents acting on ATP-dependent potassium channels of β-cells; cholestyramine, colestipol, clofibrate, gemfibrozil, lovastatin, pravastatin, simvastatin, probucol, dextrothyroxine, nateglinide, and repaglinide; β-blockers such as alprenolol, atenolol, timolol, pindolol, propranolol, and metoprolol; ACE (angiotensin-converting enzyme) inhibitors such as benazepril, captopril, enalapril, fosinopril, lisinopril, fasidotril, quinapril, and ramipril; calcium channel blockers such as nifedipine, felodipine, nicardipine, isradipine, nimodipine, diltiazem, and verapamil; α-blockers such as doxazosin, urapidil, prazosin, and terazosin; CART (cocaine- and amphetamine-regulated transcript) agonists, NPY (neuropeptide Y) antagonists, PYY agonists, Y2 receptor agonists, Y4 receptor agonists, mixed Y2/Y4 receptor agonists, MC4 (melanocortin 4) agonists, orexin antagonists, TNF (tumor necrosis factor) agonists, CRF (corticotropin-releasing factor) agonists, CRF BP (corticotropin-releasing factor-binding protein) antagonists, urocortin agonists, β3 agonists, oxyntomodulin and analogs, MSH (melanocyte-stimulating hormone) agonists, MCH (melanocyte-concentrating hormone) antagonists, CCK (cholecystokinin) agonists, serotonin reuptake inhibitors, serotonin and norepinephrine reuptake inhibitors, mixed serotonin and noradrenergic compounds, 5HT (serotonin) agonists, bombesin agonists, galanin antagonists, growth hormones, growth hormone-releasing compounds, TRH (thyrotropin-releasing hormone) agonists, UCP2 or 3 (uncoupling protein 2 or 3) modulators, leptin agonists, DA agonists (bromocriptine and doprexin), lipase/amylase inhibitors, RXR (retinoid X receptor) modulators, and TRβ agonists; histamine H3 antagonists, gastric inhibitory polypeptide agonists or antagonists (GIP analogs), gastrin and gastrin analogs.

The pharmaceutical composition of the present application may be used to prevent and/or treat a disease, preferably a metabolism-related disease, a nerve-related disease, or a brain-related disease, including but not limited to a carbohydrate metabolism disorder and/or a lipid metabolism disorder and/or a neurodegenerative disease.

In some embodiments of the present application, the carbohydrate metabolism disorder is a disease related to abnormal blood glucose, and the lipid metabolism disorder is a disease related to abnormal body weight. The disease related to abnormal blood glucose refers to a disease/symptom accompanied by abnormal blood glucose or a complication/symptom caused by a disease accompanied by abnormal blood glucose, and is selected from one or more of the following: diabetes, diabetic oculopathy, diabetic heart disease, diabetic nephropathy, diabetic neuropathy, necrosis of distal lower limbs, atherosclerosis, coronary heart disease, myocardial infarction, cerebral thrombosis, cerebral hemorrhage, cerebral embolism, osteoporosis, hyperlipidemia, hypertension, obesity, fatty liver, and cirrhosis. The diabetes includes, for example, hyperglycemia, type II diabetes, impaired glucose tolerance, type I diabetes, non-insulin-dependent diabetes, MODY (maturity-onset diabetes of the young), and gestational diabetes. The disease related to abnormal body weight refers to a disease/symptom accompanied by abnormal body weight or a complication/symptom caused by a disease accompanied by abnormal body weight, and is selected from one or more of the following: anemia, gastroptosis, inflammatory bowel disease, dyspepsia, gastrointestinal ulcer, endocrine dyscrasia, osteoporosis, obesity, hypertension, hyperlipidemia, coronary heart disease, diabetes, fatty liver, joint deformity, pain, cancer, respiratory insufficiency, kidney diseases, hyperlipidemia, atherosclerosis, cirrhosis, angina pectoris, and myocardial infarction.

In some embodiments of the present application, the neurodegenerative disease is selected from one or more of the following: brain atrophy, cerebral ischemia, brain injury, epilepsy, Parkinson's disease, Alzheimer's disease, amyotrophic lateral sclerosis, multiple sclerosis, Huntington's disease, spinocerebellar ataxia, Pick's disease, bovine spongiform encephalopathy, and Creutzfeldt-Jakob disease.

The pharmaceutical composition of the present application may also be used as a therapeutic agent for reducing HbA1C; delaying or preventing the progression of diabetes, such as the progression of type 2 diabetes, delaying the progression of impaired glucose tolerance (IGT) to insulin-requiring type 2 diabetes, and/or delaying the progression of non-insulin-requiring type 2 diabetes to insulin-requiring type 2 diabetes; improving the function of β-cells, such as reducing the apoptosis of β-cells, improving the function of β-cells and/or the mass of β-cells, and/or restoring glucose sensitivity of β-cells.

In the present application, "treat" and other similar synonyms include alleviating, relieving, or ameliorating a symptom of a disease or condition, preventing other symptoms, ameliorating or preventing the underlying metabolic causes of a symptom, inhibiting a disease or condition, e.g., arresting the progression of a disease or condition, alleviating a disease or condition, causing regression of a disease or condition, alleviating a symptom caused by a disease or condition, or stopping a symptom of a disease or condition. In addition, the term includes prophylactic purposes. The term further includes achieving therapeutic effects and/or prophylactic effects.

In the present application, "patient" refers to an individual suffering from a disease, disorder, condition, or the like, including mammals. Examples of mammals include, but are not limited to, any member of the class Mammalia: humans, non-human primates (e.g., chimpanzees and other apes and monkeys); livestock animals, such as cattle, horses, sheep, goats, and pigs; domestic animals, such as rabbits, dogs, and cats; laboratory animals, including rodents, such as rats, mice, guinea pigs, and the like.

In the present application, "C5-C11" means that the group has 5-11 carbon atoms, that is, the group contains 5 carbon atoms (C5), 6 carbon atoms (C6), 7 carbon atoms (C7), 8 carbon atoms (C8), 9 carbon atoms (C9), 10 carbon atoms (C10), or 11 carbon atoms (C11).

In the present application, a numerical range, such as "4-10" or "6-8", refers to integers in the given range, including 4, 5, 6, 7, 8, 9, or 10.

The present application will be further illustrated with reference to the following specific examples. It should be understood that the specific embodiments described herein are presented by way of example and are not intended to limit the present application. The main features of the present application can be used in various embodiments without departing from the scope of the present application.

The GLP-1 receptor agonist used in the examples and comparative examples has a structure represented by formula (I), and is named N-ε³⁰-[2-(2-[2-(2-[2-(2-[4-(17-carboxyheptadecanoylamino)-4(s)-carboxybutanoylamino]ethoxy)ethoxy]acetylamino)ethoxy]ethoxy)acetyl](Val⁸Glu²²Lys³⁰Arg^{26,3 4}-GLP-1 (7-37)) peptide. It is a glucagon-like peptide-1 receptor (GLP-1R) polypeptide agonist and was prepared with reference to WO2019201328A1.

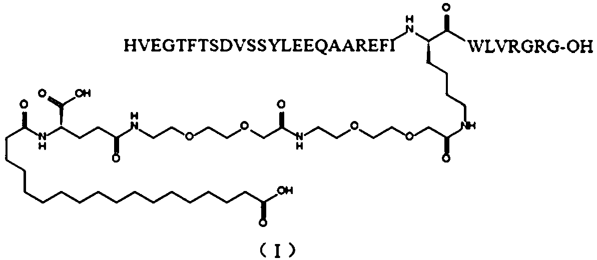

The PNAC used in the examples and comparative examples is an anhydrous crystal form sample of potassium N-(8-(2-hydroxybenzoyl)amino)caprylate, and the structure of PNAC is represented by formula (II), which was prepared with reference to CN 112661663B, i.e., crystal form I.

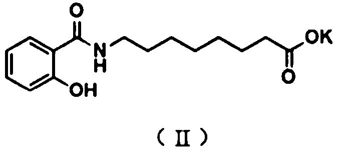

Unless otherwise specified, the test animals, test instruments, test reagents, and the like used in the examples and comparative examples are all common, commercially available products.

The analysis method for the content of the GLP-1 receptor agonist used in the examples and comparative examples is as follows:
The content of the active substance GLP-1 receptor agonist is analyzed by HPLC (General Chapter 0512, Chinese Pharmacopoeia, Volume IV, 2020 Edition). The specific procedures are as follows: 5 tablets of the composition are weighed, ground, and mixed well. A proper amount of the composition is weighed out and dissolved in a diluent (0.01% Tween 20 + 0.05 M Na₂HPO₄). After sufficient dissolution, the solution is diluted to volume, shaken uniformly, and left to stand for 30 min for sufficient extraction. After being shaken uniformly, the sample is centrifuged (at 12,000 rpm for 10 min at 4 °C), and the supernatant is added to an HPLC liquid phase vial with a proper volume. The sample is injected for analysis and detected on a Waters high-performance liquid chromatograph. The reversed-phase chromatography column is a YMC UltraHT Pro C18 column. In addition, instruments such as an electronic balance (1/100,000), an ultrasonic equipment, a volumetric flask, a reagent bottle, and a sealing film are also used. The reagents used include distilled water, acetonitrile (HPLC grade), isopropanol (HPLC grade), trifluoroacetic acid (HPLC grade), anhydrous disodium hydrogen phosphate, Tween 20, and the like. Mobile phase A is water:acetonitrile:TFA in a volume ratio of 830:170:2. Mobile phase B is acetonitrile:isopropanol:TFA in a volume ratio of 850:150:2. The content of the GLP-1 receptor agonist in the composition tablet is calculated relative to the peak area of its control sample.

### Example 1. Pharmacokinetic Study in Beagle Dogs

### 1. Preparation of composition tablets

Tablets with the same amount of the GLP-1 receptor agonist were prepared, but the contents of excipients, such as the delivery agent PNAC, a delivery synergist, and other excipients, varied. Provided in Tables 1 and 2 are the contents of the components in the different composition tablets prepared.

The specific preparation procedures of the tablets are as follows: 1. Material pretreatment: The GLP-1 receptor agonist was sieved with a 100-mesh sieve; if necessary, the GLP-1 receptor agonist was milled first and then sieved. The delivery agent PNAC was sieved with a 50-mesh sieve, and the agent that did not pass through the sieve was sieved again after grinding. The delivery synergist was treated by the same method as that of PNAC. The lubricant magnesium stearate was taken directly. 2. First mixing: A certain amount of the GLP-1 receptor agonist, the delivery agent, and the delivery synergist, and a proper amount of magnesium stearate were manually mixed well to obtain a mixed powder I. 3. Dry granulation: The mixed powder I described above was compacted into strip-shaped flakes with a suitable hardness and then milled into granules. 4. Re-mixing: The granules described above were collected, the remaining magnesium stearate was added again, and the mixture was manually mixed well. 5. Tableting: The hardness of the tablet was controlled to be in the range of 30-90 N.

**Table 1. Components of different composition tablets**

| Component | Component function | Content (mg/tablet) | | | | | | |
|---|---|---|---|---|---|---|---|---|
| | | Composition A | Composition B | Composition C1 | Composition C2 | Composition C3 | Composition C4 | Composition C5 |
| GLP-1R agonist | Active ingredient | 7 | 7 | 7 | 7 | 7 | 7 | 7 |
| PNAC | Delivery agent | 100 | 300 | 100 | 100 | 100 | 100 | 100 |
| Hydrotalcite | Delivery synergist | 0 | 0 | 100 | - | - | - | - |
| Aluminum phosphate | | - | - | - | 100 | - | - | - |
| Calcium carbonate | | - | - | - | - | 100 | - | - |
| Aluminum hydroxide | | - | - | - | - | - | 100 | - |
| Sucralfate | | - | - | - | - | - | - | 100 |
| Magnesium stearate | Lubricant | 3 | 8 | 6 | 6 | 6 | 6 | 6 |
| Total Weight | NA | 110.0 | 315.0 | 213.0 | 213.0 | 213.0 | 213.0 | 213.0 |

**Table 2. Components of composition tablets containing calcium carbonate**

| Component | Content (mg/tablet) | | |
|---|---|---|---|
| | Composition D1 | Composition D2 | Composition D3 |
| GLP-1R agonist | 7 | 7 | 7 |
| PNAC | 100 | 100 | 300 |
| Calcium carbonate | 200 | 60 | 100 |
| Magnesium stearate | 8 | 4 | 10 |
| Total Weight | 315 | 171 | 417 |

### 2. Test method and result

### 2.1 Disintegration test

The tablet disintegration test in this example was performed according to the Determination of Disintegration in General Chapter 0921, Chinese Pharmacopoeia, Volume IV, 2020 Edition, and the *in vitro* disintegration time of different composition tablets was recorded. The specific method is as follows: A hanging basket was suspended from a rack via a stainless steel shaft and immersed in a 1000 mL beaker, and the position of the hanging basket was adjusted, so that when the hanging basket was lowered to a low point, the bottom sieve was about 25 mm from the bottom of the beaker. Purified water at a temperature of 37±1 °C was contained in the beaker. The water level was adjusted, so that when the hanging basket was raised to a high point, the sieve was 15 mm below the water surface, and the top of the hanging basket was not immersed in the solution.

In this example, a ZB-1D intelligent disintegration apparatus from Tianjin Tianhe Analysis Instrument Co., Ltd. was used to measure the disintegration time according to the method. The median value of 3-6 composition tablets was taken as the result.

The results are shown in Table 3. The disintegration time of different composition tablets varied with the type or proportion of the delivery synergist, and was increased to different degrees relative to the reference composition A.

**Table 3. Disintegration time results**

| Composition | A | B | C1 | C2 | C3 | C4 | C5 | D1 | D2 | D3 |
|---|---|---|---|---|---|---|---|---|---|---|
| Disintegration time/min | 4.5 | 7.5 | 17.5 | 5.5 | 8.5 | 9.0 | 7.5 | >20 | 5.5 | 7.5 |

### 2.2 Pharmacokinetic study in beagle dogs

The beagle dogs used were male and about 0.5 to 6 years old, and weighed about 9-12 kg. Some of the beagle dogs were subjected to repeated pharmacokinetic studies. After the end of the previous round of administration, the beagle dogs were given an appropriate washout period during which their states (such as mental state, feeding state/amount, body weight state, etc.) were evaluated, and only those who passed the evaluation could enter the next round of administration experimental group.

Administration requirements are as follows: The beagle dogs were all fasted overnight (free access to water) before each administration, and administration was performed in this state; the tablets were taken with 10 mL of water (quantitative) during administration to ensure that the tablets were not chewed and remained intact, and food intake was resumed 4 h after administration. Blood samples were collected before administration (0 h) and 0.5 h, 1.5 h, 3 h, 8 h, and 24 h after administration, continuing until 24 h after administration, to fully cover the complete plasma concentration-time absorption profile of the GLP-1 receptor agonist.

Blood samples were collected at each blood collection time point. The brief preparation procedures are as follows: About 1.5 mL of whole blood was anticoagulated and then centrifuged at 5000× g for 10 min at 4 °C, and the resulting plasma was extracted and separated. The plasma was collected in EDTA anticoagulant EP tubes, with 600 µL or more per tube. The tubes were stored in a refrigerator at -80 °C until detection and analysis.

The active ingredients in the blood samples were detected by liquid chromatography-mass spectrometry (LC-MS). The mass spectrometer was a Q EXACTIVE instrument from Thermo, and the high-performance liquid chromatography was performed using an Ultimate 3000 instrument from Thermo.

Plasma concentrations of the GLP-1 receptor agonist were analyzed by using LC-MS to obtain plasma concentration-time curves. The exposure of the GLP-1 receptor agonist within 0-3 h was determined, and AUC_{(0-3 h)} and Cₘₐₓ were obtained and normalized by calculating the ratio to (administration dose (mg)/body weight (Kg)) × 100 to obtain the dose-corrected exposure.

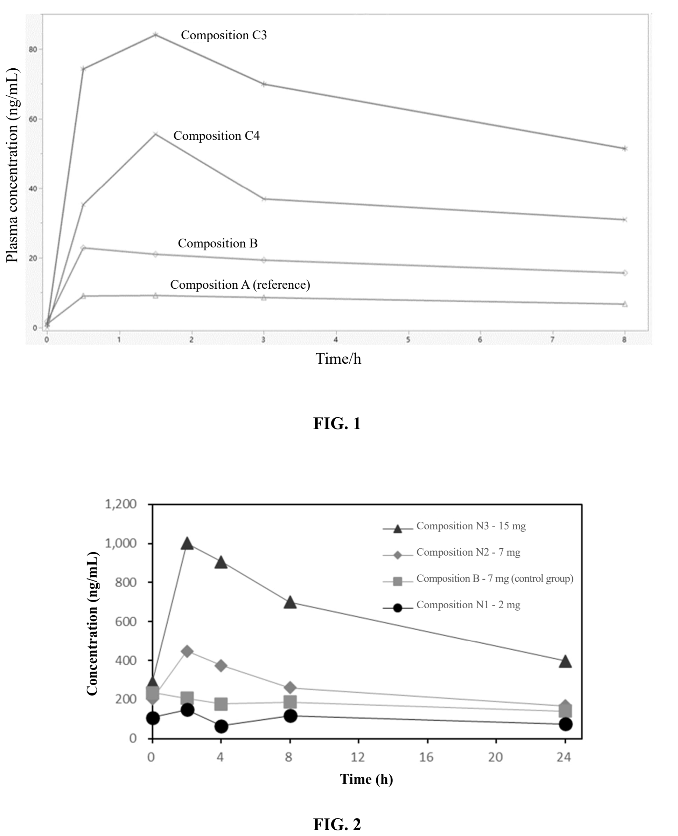

As shown in Table 4 and FIG. 1, when the amount of the delivery agent (PNAC) used was the same, an increase in exposure was observed in beagle dogs after oral administration of the composition tablets containing the delivery synergist; when the delivery synergist was not contained, the amount of the delivery agent (PNAC) used was reduced, and the exposure after oral administration was significantly reduced (Composition A) as compared with Composition B; by increasing the delivery synergist, the exposure after oral administration was significantly improved, and the overall amount of excipients used was reduced. The results show that the delivery synergist can effectively reduce the amount of the delivery agent used and improve the oral bioavailability of the polypeptide.

**Table 4. Evaluation results of in vivo exposure of the GLP-1 receptor agonist in the pharmacokinetic study**

| Composition | Dose-corrected AUC 0-3 h ((Kg*h/L)*100) | Dose-corrected exposure maximum within 0-3 h ((Kg/L)*100) |
|---|---|---|
| Composition A (control group) | 3.6 | 1.31 |
| Composition B | 8.3 | 3.26 |
| Composition C1 | 15.4 | 6.5 |
| Composition C2 | 24.3 | 9.5 |
| Composition C3 | 33.8 | 13.3 |
| Composition C4 | 19.8 | 8.9 |
| Composition C5 | 12.7 | 5.7 |
| Composition D1 | 17.7 | 7.9 |
| Composition D2 | 29.2 | 11.4 |
| Composition D3 | 36.0 | 13.5 |

### Example 2. Steady-State Pharmacokinetic Study in Beagle Dogs

### 1. Preparation of composition tablets

Provided in Table 5 are the contents of the components in different composition tablets (Compositions M1-M9) used in this example. The preparation process of the tablet was direct mixing followed by tableting. The specific procedures are as follows: 1. Material pretreatment: The GLP-1R agonist was sieved with a 100-mesh sieve; if necessary, the GLP-1R agonist was milled first and then sieved. The delivery agent PNAC was sieved with a 50-mesh sieve, and the agent that did not pass through the sieve was sieved again after grinding. Other excipients were all sieved with a 40-mesh sieve. 2. Mixing: A certain amount of the GLP-1R agonist, the delivery agent, the delivery synergist, and other excipients were manually mixed well to obtain a mixed powder I. 3. Tableting: Tableting was performed according to requirements, and the hardness of the tablet was controlled to be in the range of 30-90 N.

**Table 5. Components of composition tablets for the steady-state pharmacokinetic study in beagle dogs**

| Component | Content (mg/tablet) | | | | | | | | |
|---|---|---|---|---|---|---|---|---|---|
| | Composition M1 | Composition M2 | Composition M3 | Composition M4 | Composition M5 | Composition M6 | Composition M7 | Composition M8 | Composition M9 |
| GLP-1R agonist | 7 | 7 | 7 | 7 | 7 | 7 | 7 | 7 | 7 |
| PNAC | 100 | 150 | 100 | 50 | 125 | 200 | 50 | 200 | 200 |
| Calcium carbonate | 100 | 100 | 100 | 50 | 125 | 200 | 200 | 50 | 200 |
| Mannitol | 100 | 50 | / | 50 | 75 | 50 | 50 | 50 | 100 |
| Microcrystalline cellulose | / | / | 150 | / | / | / | / | / | / |
| Croscarmellose sodium | 30 | 30 | 15 | 9 | 20 | 27 | 18 | 18 | 30 |
| Magnesium stearate | 8 | 8 | 5 | 4 | 9 | 12 | 8 | 8 | 13 |
| Total Weight | 345 | 345 | 377 | 170 | 360 | 496 | 333 | 333 | 550 |

### 2. Test method and result

The steady-state (multiple administrations) pharmacokinetic study was performed in beagle dogs to determine the *in vivo* exposure of the GLP-1R agonist after multiple consecutive oral administrations of different proportions of composition tablets.

The test animals and methods were the same as those in Section 2.2 of Example 1, except that the administration was performed once daily, with 1 tablet each time, for seven consecutive days, and blood samples were collected before administration (0 h) and 0.5 h, 1.5 h, 3 h, 8 h, and 24 h after administration on the seventh day (Day 7).

Plasma concentrations of the GLP-1R agonist were analyzed by using LC-MS to obtain plasma concentration-time curves. The exposure of the GLP-1R agonist in animals at steady state (Day 7) within 0-24 h, i.e., AUC_{(0-24 h)} and Cₘₐₓ, was obtained and normalized by calculating the ratio to (administration dose (mg)/body weight (Kg)) × 100 to obtain the dose-corrected exposure. The results are shown in Table 6.

**Table 6. Steady-state pharmacokinetic study results in beagle dogs**

| Composition | Dose-corrected exposure maximum within 0-24 h ((Kg/L)* 100) | Multiple of exposure to that of control group | Dose-corrected AUC _{0-24 h} ((Kg*h/L)*100) | Multiple of AUC_{0-24 h} to that of control group |
|---|---|---|---|---|
| Composition B (control group) | 6.5 | 1.0 | 127.0 | 1.0 |
| Composition M1 | 15.8 | 2.4 | 266.3 | 2.1 |
| Composition M2 | 7.6 | 1.2 | 140.1 | 1.1 |
| Composition M3 | 10.2 | 1.6 | 182.8 | 1.4 |
| Composition M4 | 17.2 | 2.6 | 247.7 | 2.0 |
| Composition M5 | 17.8 | 2.7 | 256.7 | 2.0 |
| Composition M6 | 13.7 | 2.1 | 213.6 | 1.7 |
| Composition M7 | 11.6 | 1.8 | 183.7 | 1.4 |
| Composition M8 | 13.5 | 2.1 | 224.5 | 1.8 |
| Composition M9 | 6.6 | 1.0 | 211.3 | 1.7 |

The results show that compared with Composition B (control group) not containing the delivery synergist, after multiple oral administrations, the oral absorption (AUC_{0-24 h}) of Compositions M1-M9 in which the delivery synergist was mixed with PNAC at different contents and ratios was improved to different degrees at steady state. In another aspect, compared with Composition B (control group) with the amount of PNAC used up to 300 mg, the oral absorption (AUC_{0-24 h}) of the Composition M4 group with the PNAC content of only 50 mg could still be improved by 2 times due to the increased delivery synergist.

### Example 3. Pharmacokinetic Study in Cynomolgus Monkeys

### 1. Preparation of composition tablets

Provided in Table 7 are the contents of the components in different composition tablets (Compositions N1-N3) used in this example. The preparation process of the tablets was the same as that in Section 1 of Example 1, except that mannitol and croscarmellose sodium were sieved with a 40-mesh sieve in the "Pretreatment" stage, and mixed with the granules obtained by dry granulation and the remaining magnesium stearate in the "Re-mixing" stage.

**Table 7. Components of compositions for the pharmacokinetic study in cynomolgus monkeys**

| Component | Content (mg/tablet) | | |
|---|---|---|---|
| | Composition N1 | Composition N2 | Composition N3 |
| GLP-1R agonist | 2 | 7 | 15 |
| PNAC | 100 | 100 | 100 |
| Calcium carbonate | 100 | 100 | 100 |
| Mannitol | 100 | 100 | 100 |
| Croscarmellose sodium | 16 | 16 | 16 |
| Magnesium stearate | 8 | 8 | 8 |
| Total Weight | 326 | 331 | 339 |

### 2. Test method and result

The pharmacokinetic study on different doses of composition tablets was performed in cynomolgus monkeys to determine the *in vivo* exposure of different doses of GLP-1R agonists after multiple consecutive oral administrations of different composition tablets.

In this test, a total of 12 animals (all male and weighing about 3-6 kg) were used and divided into 4 groups of 3. Oral administration: Composition N1 (2 mg), Composition N2, Composition B (7 mg), and Composition N3 (15 mg) were given. Multiple administrations were performed once daily, with 1 tablet each time, for 7 consecutive days. Plasma samples were collected from each group before administration (0 h) and 2 h, 4 h, 8 h, and 24 h after administration on the seventh day (Day 7).

Blood samples were processed according to the test method in Example 2, and the pharmacokinetic study was performed in cynomolgus monkeys after multiple administrations. Plasma concentrations of the GLP-1R agonists were analyzed to obtain plasma concentration-time curves, so that the exposure of the GLP-1R agonist in animals at steady state (Day 7) within 0-24 h, i.e., AUC_{(0-24 h)} and Cₘₐₓ, was obtained and normalized by calculating the ratio to (administration dose (mg)/body weight (Kg)) × 100 to obtain the dose-corrected exposure. The results are shown in Table 8 and FIG. 2.

**Table 8. Pharmacokinetic study results in cynomolgus monkeys**

| Group | Composition B (control group) | Composition N3 | Composition N2 | Composition N1 |
|---|---|---|---|---|
| Cₘₐₓ (ng/mL) | 235.77 | 1002.02 | 447.79 | 148.14 |
| AUC_{0-24 h}(ng/mL*h) | 4188.9 | 15191.2 | 6178.6 | 2385.1 |
| Corrected Cmax ((Kg/L)* 100) | 13.5 | 26 | 24.2 | 28.9 |
| Corrected AUC 0-24 h ((Kg*h/L)*100) | 239.4 | 394.5 | 334.1 | 465.5 |

The results show that after oral administration of Compositions N1, N2, and N3 to cynomolgus monkeys for 7 consecutive days, the systemic exposure (Cₘₐₓ and AUC_{(0-24 h)}) of the GLP-1R agonist in the cynomolgus monkeys increased with dose in a dose-dependent manner. The administration groups of Compositions N1-N3 showed similar corrected Cₘₐₓ and corrected AUC₀₋₂₄ values, which were significantly higher than those of Composition B. Compared with the 7 mg/tablet dose group, the oral absorption of Composition B without the delivery synergist was significantly lower than that of Composition N2 with the delivery synergist, and the Composition N2 group showed a significantly higher corrected AUC_{0-24 h} value, indicating that the composition containing the delivery synergist has better oral absorption.

### Example 4. Comparative Study on Different Delivery Agents

### 1. Preparation of composition tablets

In order to further verify the oral synergistic effect of the delivery synergist on the oral delivery agent, the oral effects of compositions (Compositions J1-J4) containing different oral delivery agents, sodium caprylate (C8Na), sodium caprate (C10Na), sodium laurate (C12Na), and sodium chenodeoxycholate, and the delivery synergist were investigated. The components of the compositions are shown in Table 9. The preparation method for the tablets was the same as in Example 2, i.e., direct mixing and then tableting.

**Table 9. Components of compositions containing different delivery agents in the comparative study**

| Component | Content (mg/tablet) | | | | | |
|---|---|---|---|---|---|---|
| | Composition B | Composition M1 | Composition J1 | Composition J2 | Composition J3 | Composition J4 |
| GLP-1 agonist | 7 | 7 | 7 | 7 | 7 | 7 |
| PNAC | 300 | 100 | / | / | / | / |
| C8Na | / | / | 100 | / | / | / |
| C10Na | / | / | / | 100 | / | / |
| C12Na | / | / | / | / | 100 | / |
| Sodium chenodeoxycholate | / | / | / | / | / | 100 |
| Calcium carbonate | / | 100 | 100 | 100 | 100 | 100 |
| Mannitol | / | 100 | 100 | 100 | 100 | 100 |
| Croscarmellose sodium | / | 30 | 30 | 30 | 30 | 30 |
| Magnesium stearate | 8 | 8 | 8 | 8 | 8 | 8 |
| Total Weight | 315 | 345 | 345 | 345 | 345 | 345 |

### 2. Test method and result

The pharmacokinetic study in beagle dogs was performed according to the test method in Example 2.

**Table 10. Results of the comparative study on different delivery agents**

| Composition | Dose-corrected exposure maximum within 0-24 h ((Kg/L)*100) | Dose-corrected AUC 0-24 h ((Kg*h/L)*100) |
|---|---|---|
| Composition B | 6.1 | 108.5 |
| Composition M1 | 15.7 | 257.7 |
| Composition J1 | 8.2 | 156.8 |
| Composition J2 | 5.3 | 63.8 |
| Composition J3 | 2.2 | 42.8 |
| Composition J4 | 2.7 | 45.7 |

The results of oral absorption (AUC_{0-24 h}) are shown in Table 10. Compared with the Composition B control group, although the exposure of Compositions J2-J4 was reduced to different degrees, the contents of their oral delivery agents were reduced by up to two-thirds. Surprisingly, compared with Composition B, Composition J1, like Composition M1, exhibited an increase in oral exposure when the content of the delivery agent was reduced. This indicates that the delivery synergist, calcium carbonate, also has a good synergetic effect on other delivery agents (e.g., sodium caprylate, sodium caprate, sodium laurate, sodium chenodeoxycholate, etc.).

### Comparative Example. Comparative Study on Different Carrier Agents

### 1. Preparation of composition tablets

The oral effects of composition tablets (Compositions F1-F7) prepared by compounding several other agents having a potential delivery synergetic effect with the delivery agent PNAC were investigated. The components of the compositions are shown in Table 11. The preparation method for the tablets was the same as in Example 1.

**Table 11. Components of compositions in the comparative example**

| Component | Content (mg/tablet) | | | | | | | | |
|---|---|---|---|---|---|---|---|---|---|
| | Composition A | Composition B | F1 | F2 | F3 | F4 | F5 | F6 | F7 |
| GLP-1R agonist | 7 | 7 | 7 | 7 | 7 | 7 | 7 | 7 | 7 |
| PNAC | 100 | 300 | 100 | 100 | 100 | 300 | 100 | 100 | 100 |
| Magnesium aluminum silicate | - | - | 100 | - | - | - | - | - | - |
| Magnesium trisilicate | - | - | - | 100 | - | - | - | - | - |
| Magnesium oxide | - | - | - | - | 100 | - | - | - | - |
| Potassium bicarbonate | - | - | - | - | - | 100 | - | - | - |
| Sodium carbonate | - | - | - | - | - | - | 100 | - | - |
| Sodium bicarbonate | - | - | - | - | - | - | - | 100 | - |
| Magnesium carbonate | - | - | - | - | - | - | - | - | 100 |
| Magnesium stearate | 3 | 8 | 6 | 6 | 6 | 6 | 6 | 6 | 6 |
| Total Weight | 110 | 315 | 213 | 213 | 213 | 213 | 213 | 213 | 213 |

### 2. Test method and result

The pharmacokinetic test in beagle dogs was performed according to the test method in Example 1, and the results are shown in Table 12.

**Table 12. In vivo exposure results of the GLP-1 receptor agonist evaluated in the pharmacokinetic study of the comparative example**

| Composition | Dose-corrected AUC 0-3 h ((Kg*h/L)*100) | Dose-corrected exposure maximum within 0-3 h ((Kg/L)* 100) |
|---|---|---|
| Composition A (control group) | 3.6 | 1.31 |
| Composition B | 8.3 | 3.26 |
| F1 | 1.54 | 0.57 |
| F2 | 0.45 | 0.20 |
| F3 | 0.57 | 0.30 |
| F4 | 2.02 | 0.92 |
| F5 | 2.31 | 1.04 |
| F6 | 2.29 | 1.00 |
| F7 | 1.28 | 0.52 |

The results show that when no delivery synergist was contained, Composition A had a reduced amount of the delivery agent (PNAC) used and exhibited significantly reduced oral exposure compared with Composition B; when the same amount of the delivery agent (PNAC) was used, the composition tablets containing several candidate synergists selected in this comparative example and the delivery agent exhibited reduced oral exposure in beagle dogs. The results show that although certain structural features of the candidate synergists in this comparative example were similar to those of the delivery synergists of the present application, oral administration of the composition tablets containing several candidate synergists in this comparative example could not improve the drug exposure, but reduced the drug exposure, indicating that not all similar carrier agents commonly used in the pharmaceutical field can reduce the amount of the delivery agent used and improve the bioavailability of the polypeptide.

The foregoing examples are merely intended to understand the method and core idea of the present application. It should be noted that for those of ordinary skill in the art, without departing from the principle of the present application, several improvements and modifications can also be made to the present application, and these improvements and modifications also fall within the protection scope of the claims of the present application.

## Claims

1. Use of a delivery synergist in improving the oral bioavailability of a protein or polypeptide drug, wherein the protein or polypeptide drug is used in combination with the delivery synergist, and the delivery synergist comprises hydrotalcite, aluminum phosphate, calcium carbonate, aluminum hydroxide, sucralfate, calcium bicarbonate, aluminum bicarbonate, magnesium bicarbonate, and/or magnesium hydroxide;
the protein or polypeptide drug is delivered by a delivery agent;
preferably, the delivery synergist comprises hydrotalcite, aluminum phosphate, calcium carbonate, aluminum hydroxide, and/or sucralfate.

2. The use according to claim 1, wherein the protein or polypeptide drug comprises a hormone, a growth factor, a cytokine, an analgesic peptide, an enzyme, a coagulation factor, a peptide neurotransmitter, and/or an antibody.

3. The use according to claim 1, wherein the protein or polypeptide drug comprises a receptor agonist or antagonist;
preferably, the receptor agonist or antagonist comprises a GLP-1 receptor agonist, a GLP-1 receptor antagonist, a GIP receptor agonist, a GIP receptor antagonist, and/or an insulin receptor agonist;
preferably, the receptor agonist or antagonist comprises a GLP-1 receptor agonist;
preferably, the GLP-1 receptor agonist comprises an exendin, GLP-1 or an analog, fragment, derivative, fusion protein, or conjugate thereof, or a pharmaceutically acceptable salt of the molecule described above;
preferably, the GLP-1 receptor agonist has a structure represented by formula (I):

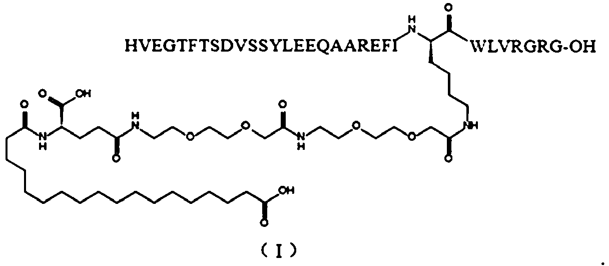

4. The use according to any one of claims 1-3, wherein the delivery agent has a structure represented by formula (III): 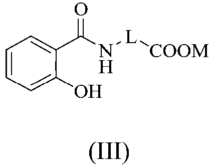
in formula (III), L represents linear or branched C5-C11 alkylene, and M represents Na⁺, K⁺, or NH₄⁺;
preferably, L represents linear or branched C7-C9 alkylene, and M represents K⁺;
preferably, the delivery agent comprises a salt of N-(8-(2-hydroxybenzoyl)amino)caprylic acid;
preferably, the salt of N-(8-(2-hydroxybenzoyl)amino)caprylic acid comprises a sodium salt, a potassium salt, and/or an ammonium salt;
preferably, the salt of N-(8-(2-hydroxybenzoyl)amino)caprylic acid is selected from a potassium salt, which has a structure represented by formula (II): 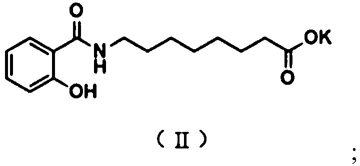
or the delivery agent has a structure represented by formula (IV):
CH₃-(CH₂)n-COOM, (IV)
in formula (IV), n represents an integer from 4 to 10, and M represents Na⁺, K⁺, or NH₄⁺;
preferably, n represents an integer from 6 to 8, and M represents Na⁺;
preferably, the delivery agent comprises a salt of caprylic acid, capric acid, and/or lauric acid;
preferably, the delivery agent comprises a sodium salt, a potassium salt, and/or an ammonium salt of caprylic acid, capric acid, and/or lauric acid;
or the delivery agent comprises a salt of chenodeoxycholic acid;
preferably, the delivery agent comprises a sodium salt, a potassium salt, and/or an ammonium salt of chenodeoxycholic acid;
preferably, the delivery agent and the delivery synergist are in a mass ratio of 1:0.3-1:3;
preferably, the delivery agent and the delivery synergist are in a mass ratio of 1:0.3-1:2;
preferably, the combination use comprises simultaneously using the protein or polypeptide drug and the delivery synergist in the form of a pharmaceutical composition; or separately using the protein or polypeptide drug and the delivery synergist in sequence;
preferably, the combination use is to simultaneously use the protein or polypeptide drug and the delivery synergist in the form of a pharmaceutical composition;
preferably, the protein or polypeptide drug and the delivery agent are in a mass ratio of 1:5-1:150;
preferably, the protein or polypeptide drug and the delivery agent are in a mass ratio of 1:10-1:50;
preferably, the pharmaceutical composition further comprises a pharmaceutically acceptable carrier;
preferably, the pharmaceutically acceptable carrier comprises a lubricant, a filler, a disintegrant, a flavoring agent, a colorant, a binder, an forming agent, a buffer, a diluent, a solubilizer, a tonicity regulator, a surfactant, a preservative, an isotonic agent, a stabilizer, and/or a chelating agent;
preferably, the pharmaceutically acceptable carrier comprises a lubricant, a filler, and a disintegrant;
preferably, the lubricant comprises magnesium stearate, glyceryl tribehenate, and/or stearic acid;
preferably, the lubricant is selected from magnesium stearate;
preferably, the filler is selected from mannitol and/or microcrystalline cellulose;
preferably, the disintegrant is selected from croscarmellose sodium;
preferably, the pharmaceutical composition further comprises one or more active pharmaceutical ingredients for use in combination with the protein or polypeptide drug;
preferably, the active pharmaceutical ingredient is selected from an antidiabetic agent, an anti-obesity agent, an appetite-regulating agent, an antihypertensive agent, an agent for treating and/or preventing complications and conditions caused by or associated with diabetes, and an agent for treating and/or preventing complications and conditions caused by or associated with obesity;
preferably, a dosage form of the pharmaceutical composition comprises a tablet, a capsule, an emulsion, an aqueous suspension, a dispersant, or a powder;
preferably, the dosage form of the pharmaceutical composition is a tablet;
preferably, the tablet is prepared by a dry granulation process and/or a direct mixing process;
preferably, the tablet has a hardness of 30-90 N.

5. A method for improving the oral bioavailability of a protein or polypeptide drug, comprising:
using the protein or polypeptide drug in combination with a delivery synergist, wherein the delivery synergist comprises hydrotalcite, aluminum phosphate, calcium carbonate, aluminum hydroxide, sucralfate, calcium bicarbonate, aluminum bicarbonate, magnesium bicarbonate, and/or magnesium hydroxide;
the protein or polypeptide drug is delivered by a delivery agent;
preferably, the delivery synergist comprises hydrotalcite, aluminum phosphate, calcium carbonate, aluminum hydroxide, and/or sucralfate.

6. The method according to claim 5, wherein the protein or polypeptide drug comprises a hormone, a growth factor, a cytokine, an analgesic peptide, an enzyme, a coagulation factor, a peptide neurotransmitter, and/or an antibody.

7. The method according to claim 5, wherein the protein or polypeptide drug comprises a receptor agonist or antagonist;
preferably, the receptor agonist or antagonist comprises a GLP-1 receptor agonist, a GLP-1 receptor antagonist, a GIP receptor agonist, a GIP receptor antagonist, and/or an insulin receptor agonist;
preferably, the receptor agonist or antagonist comprises a GLP-1 receptor agonist;
preferably, the GLP-1 receptor agonist comprises an exendin, GLP-1 or an analog, fragment, derivative, fusion protein, or conjugate thereof, or a pharmaceutically acceptable salt of the molecule described above;
preferably, the GLP-1 receptor agonist has a structure represented by formula (I):

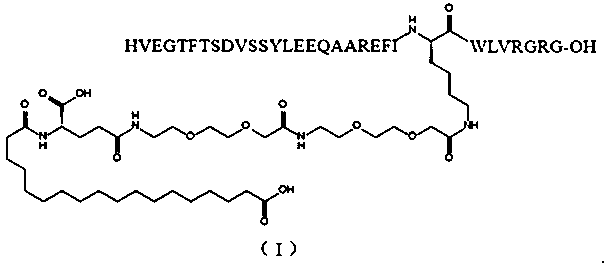

8. The method according to any one of claims 5-7, wherein the combination use comprises simultaneously using the protein or polypeptide drug and the delivery synergist in the form of a pharmaceutical composition; or separately using the protein or polypeptide drug and the delivery synergist in sequence;
preferably, the combination use is to simultaneously use the protein or polypeptide drug and the delivery synergist in the form of a pharmaceutical composition;
preferably, a dosage form of the pharmaceutical composition comprises a tablet, a capsule, an emulsion, an aqueous suspension, a dispersant, or a powder;
preferably, the dosage form of the pharmaceutical composition is a tablet;
preferably, the tablet is prepared by a dry granulation process and/or a direct mixing process;
preferably, the tablet has a hardness of 30-90 N.

9. The method according to any one of claims 5-8, wherein the delivery agent has a structure represented by formula (III): 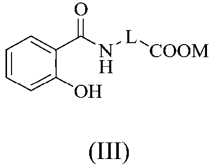
in formula (III), L represents linear or branched C5-C11 alkylene, and M represents Na⁺, K⁺, or NH₄⁺;
preferably, L represents linear or branched C7-C9 alkylene, and M represents K⁺;
preferably, the delivery agent comprises a salt of N-(8-(2-hydroxybenzoyl)amino)caprylic acid;
preferably, the salt of N-(8-(2-hydroxybenzoyl)amino)caprylic acid comprises a sodium salt, a potassium salt, and/or an ammonium salt;
preferably, the salt of N-(8-(2-hydroxybenzoyl)amino)caprylic acid is selected from a potassium salt, which has a structure represented by formula (II): 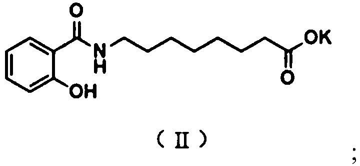
or the delivery agent has a structure represented by formula (IV):
CH₃-(CH₂)n-COOM, (IV)
in formula (IV), n represents an integer from 4 to 10, and M represents Na⁺, K⁺, or NH₄⁺;
preferably, n represents an integer from 6 to 8, and M represents Na⁺;
preferably, the delivery agent comprises a salt of caprylic acid, capric acid, and/or lauric acid;
preferably, the delivery agent comprises a sodium salt, a potassium salt, and/or an ammonium salt of caprylic acid, capric acid, and/or lauric acid;
or the delivery agent comprises a salt of chenodeoxycholic acid;
preferably, the delivery agent comprises a sodium salt, a potassium salt, and/or an ammonium salt of chenodeoxycholic acid;
preferably, the delivery agent and the delivery synergist are in a mass ratio of 1:0.3-1:3;
preferably, the delivery agent and the delivery synergist are in a mass ratio of 1:0.3-1:2;
preferably, the protein or polypeptide drug and the delivery agent are in a mass ratio of 1:5-1:150;
preferably, the protein or polypeptide drug and the delivery agent are in a mass ratio of 1:10-1:50.

10. The method according to claim 8, wherein the pharmaceutical composition further comprises a pharmaceutically acceptable carrier;
preferably, the pharmaceutically acceptable carrier comprises a lubricant, a filler, a disintegrant, a flavoring agent, a colorant, a binder, an forming agent, a buffer, a diluent, a solubilizer, a tonicity regulator, a surfactant, a preservative, an isotonic agent, a stabilizer, and/or a chelating agent;
preferably, the pharmaceutically acceptable carrier comprises a lubricant, a filler, and a disintegrant;
preferably, the lubricant comprises magnesium stearate, glyceryl tribehenate, and/or stearic acid;
preferably, the lubricant is selected from magnesium stearate;
preferably, the filler is selected from mannitol and/or microcrystalline cellulose;
preferably, the disintegrant is selected from croscarmellose sodium;
preferably, the pharmaceutical composition further comprises one or more active pharmaceutical ingredients for use in combination with the protein or polypeptide drug;
preferably, the active pharmaceutical ingredient is selected from an antidiabetic agent, an anti-obesity agent, an appetite-regulating agent, an antihypertensive agent, an agent for treating and/or preventing complications and conditions caused by or associated with diabetes, and an agent for treating and/or preventing complications and conditions caused by or associated with obesity.

11. A pharmaceutical composition having improved oral bioavailability and having a protein or polypeptide drug as an active ingredient, comprising a delivery synergist, wherein the delivery synergist comprises hydrotalcite, aluminum phosphate, calcium carbonate, aluminum hydroxide, sucralfate, calcium bicarbonate, aluminum bicarbonate, magnesium bicarbonate, and/or magnesium hydroxide;
the pharmaceutical composition further comprises a delivery agent;
preferably, the delivery synergist comprises hydrotalcite, aluminum phosphate, calcium carbonate, aluminum hydroxide, and/or sucralfate;
preferably, the protein or polypeptide drug comprises a hormone, a growth factor, a cytokine, an analgesic peptide, an enzyme, a coagulation factor, a peptide neurotransmitter, and/or an antibody;
or the protein or polypeptide drug comprises a receptor agonist or antagonist;
preferably, the receptor agonist or antagonist comprises a GLP-1 receptor agonist, a GLP-1 receptor antagonist, a GIP receptor agonist, a GIP receptor antagonist, and/or an insulin receptor agonist;
preferably, the receptor agonist or antagonist comprises a GLP-1 receptor agonist;
preferably, the GLP-1 receptor agonist comprises an exendin, GLP-1 or an analog, fragment, derivative, fusion protein, or conjugate thereof, or a pharmaceutically acceptable salt of the molecule described above;
preferably, the GLP-1 receptor agonist has a structure represented by formula (I): 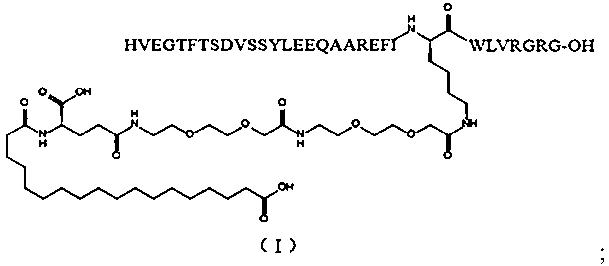
preferably, the delivery agent has a structure represented by formula (III): 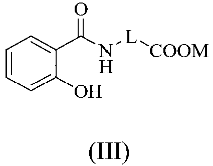
in formula (III), L represents linear or branched C5-C11 alkylene, and M represents Na⁺, K⁺, or NH₄⁺;
preferably, L represents linear or branched C7-C9 alkylene, and M represents K⁺;
preferably, the delivery agent comprises a salt of N-(8-(2-hydroxybenzoyl)amino)caprylic acid;
preferably, the salt of N-(8-(2-hydroxybenzoyl)amino)caprylic acid comprises a sodium salt, a potassium salt, and/or an ammonium salt;
preferably, the salt of N-(8-(2-hydroxybenzoyl)amino)caprylic acid is selected from a potassium salt, which has a structure represented by formula (II): 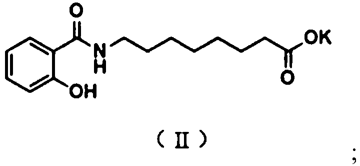
or the delivery agent has a structure represented by formula (IV):
CH₃-(CH₂)n-COOM, (IV)
in formula (IV), n represents an integer from 4 to 10, and M represents Na⁺, K⁺, or NH₄⁺;
preferably, n represents an integer from 6 to 8, and M represents Na⁺;
preferably, the delivery agent comprises a salt of caprylic acid, capric acid, and/or lauric acid;
preferably, the delivery agent comprises a sodium salt, a potassium salt, and/or an ammonium salt of caprylic acid, capric acid, and/or lauric acid;
or the delivery agent comprises a salt of chenodeoxycholic acid;
preferably, the delivery agent comprises a sodium salt, a potassium salt, and/or an ammonium salt of chenodeoxycholic acid;
preferably, the delivery agent and the delivery synergist are in a mass ratio of 1:0.3-1:3;
preferably, the delivery agent and the delivery synergist are in a mass ratio of 1:0.3-1:2;
preferably, the protein or polypeptide drug and the delivery agent are in a mass ratio of 1:5-1:150;
preferably, the protein or polypeptide drug and the delivery agent are in a mass ratio of 1:10-1:50;
preferably, the protein or polypeptide drug is used in combination with the delivery synergist;
preferably, the combination use comprises simultaneously using the protein or polypeptide drug and the delivery synergist in the form of a pharmaceutical composition; or separately using the protein or polypeptide drug and the delivery synergist in sequence;
preferably, the combination use is to simultaneously use the protein or polypeptide drug and the delivery synergist in the form of a pharmaceutical composition.

12. The pharmaceutical composition according to claim 11, further comprising a pharmaceutically acceptable carrier;
preferably, the pharmaceutically acceptable carrier comprises a lubricant, a filler, a disintegrant, a flavoring agent, a colorant, a binder, an forming agent, a buffer, a diluent, a solubilizer, a tonicity regulator, a surfactant, a preservative, an isotonic agent, a stabilizer, and/or a chelating agent;
preferably, the pharmaceutically acceptable carrier comprises a lubricant, a filler, and a disintegrant;
preferably, the lubricant comprises magnesium stearate, glyceryl tribehenate, and/or stearic acid;
preferably, the lubricant is selected from magnesium stearate;
preferably, the filler is selected from mannitol and/or microcrystalline cellulose;
preferably, the disintegrant is selected from croscarmellose sodium;
preferably, the pharmaceutical composition further comprises one or more active pharmaceutical ingredients for use in combination with the protein or polypeptide drug;
preferably, the active pharmaceutical ingredient is selected from an antidiabetic agent, an anti-obesity agent, an appetite-regulating agent, an antihypertensive agent, an agent for treating and/or preventing complications and conditions caused by or associated with diabetes, and an agent for treating and/or preventing complications and conditions caused by or associated with obesity;
preferably, a dosage form of the pharmaceutical composition comprises a tablet, a capsule, an emulsion, an aqueous suspension, a dispersant, or a powder;
preferably, the dosage form of the pharmaceutical composition is a tablet;
preferably, the tablet is prepared by a dry granulation process and/or a direct mixing process;
preferably, the tablet has a hardness of 30-90 N.

13. Use of a substance shown below as a synergist for a drug delivery agent: hydrotalcite, aluminum phosphate, calcium carbonate, aluminum hydroxide, sucralfate, calcium bicarbonate, aluminum bicarbonate, magnesium bicarbonate, and/or magnesium hydroxide;
wherein the delivery agent has a structure represented by formula (III): 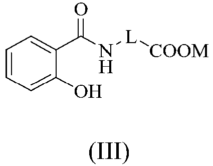
in formula (III), L represents linear or branched C5-C11 alkylene, and M represents Na⁺, K⁺, or NH₄⁺;
preferably, L represents linear or branched C7-C9 alkylene, and M represents K⁺;
preferably, the delivery agent comprises a salt of N-(8-(2-hydroxybenzoyl)amino)caprylic acid;
preferably, the salt of N-(8-(2-hydroxybenzoyl)amino)caprylic acid comprises a sodium salt, a potassium salt, and/or an ammonium salt;
preferably, the salt of N-(8-(2-hydroxybenzoyl)amino)caprylic acid is selected from a potassium salt, which has a structure represented by formula (II): 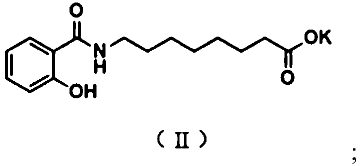
or the delivery agent has a structure represented by formula (IV):
CH₃-(CH₂)n-COOM, (IV)
in formula (IV), n represents an integer from 4 to 10, and M represents Na⁺, K⁺, or NH₄⁺;
preferably, n represents an integer from 6 to 8, and M represents Na⁺;
preferably, the delivery agent comprises a salt of caprylic acid, capric acid, and/or lauric acid;
preferably, the delivery agent comprises a sodium salt, a potassium salt, and/or an ammonium salt of caprylic acid, capric acid, and/or lauric acid;
or the delivery agent comprises a salt of chenodeoxycholic acid;
preferably, the delivery agent comprises a sodium salt, a potassium salt, and/or an ammonium salt of chenodeoxycholic acid.

14. A pharmaceutical composition, comprising a delivery agent and a delivery synergist, wherein the delivery agent has a structure represented by formula (III): 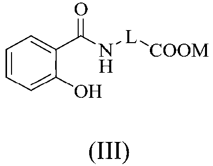
in formula (III), L represents linear or branched C5-C11 alkylene, and M represents Na⁺, K⁺, or NH₄⁺;
preferably, L represents linear or branched C7-C9 alkylene, and M represents K⁺;
preferably, the delivery agent comprises a salt of N-(8-(2-hydroxybenzoyl)amino)caprylic acid; preferably, the salt of N-(8-(2-hydroxybenzoyl)amino)caprylic acid comprises a sodium salt, a potassium salt, and/or an ammonium salt;
preferably, the salt of N-(8-(2-hydroxybenzoyl)amino)caprylic acid is selected from a potassium salt, which has a structure represented by formula (II): 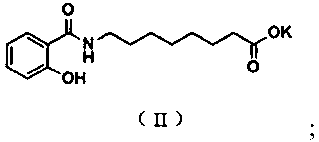
or the delivery agent has a structure represented by formula (IV):
CH₃-(CH₂)n-COOM, (IV)
in formula (IV), n represents an integer from 4 to 10, and M represents Na⁺, K⁺, or NH₄⁺;
preferably, n represents an integer from 6 to 8, and M represents Na⁺;
preferably, the delivery agent comprises a salt of caprylic acid, capric acid, and/or lauric acid;
preferably, the delivery agent comprises a sodium salt, a potassium salt, and/or an ammonium salt of caprylic acid, capric acid, and/or lauric acid;
or the delivery agent comprises a salt of chenodeoxycholic acid;
preferably, the delivery agent comprises a sodium salt, a potassium salt, and/or an ammonium salt of chenodeoxycholic acid;
the delivery synergist comprises hydrotalcite, aluminum phosphate, calcium carbonate, aluminum hydroxide, sucralfate, calcium bicarbonate, aluminum bicarbonate, magnesium bicarbonate, and/or magnesium hydroxide;
preferably, the delivery synergist comprises hydrotalcite, aluminum phosphate, calcium carbonate, aluminum hydroxide, and/or sucralfate;
preferably, the delivery agent and the delivery synergist are in a mass ratio of 1:0.3-1:3;
preferably, the delivery agent and the delivery synergist are in a mass ratio of 1:0.3-1:2.

15. The pharmaceutical composition according to claim 14, further comprising a protein or polypeptide drug, wherein the protein or polypeptide drug comprises a hormone, a growth factor, a cytokine, an analgesic peptide, an enzyme, a coagulation factor, a peptide neurotransmitter, and/or an antibody;
or the protein or polypeptide drug comprises a receptor agonist or antagonist;
preferably, the receptor agonist or antagonist comprises a GLP-1 receptor agonist, a GLP-1 receptor antagonist, a GIP receptor agonist, a GIP receptor antagonist, and/or an insulin receptor agonist;
preferably, the receptor agonist or antagonist comprises a GLP-1 receptor agonist;
preferably, the GLP-1 receptor agonist comprises an exendin, GLP-1 or an analog, fragment, derivative, fusion protein, or conjugate thereof, or a pharmaceutically acceptable salt of the molecule described above;
preferably, the GLP-1 receptor agonist has a structure represented by formula (I): 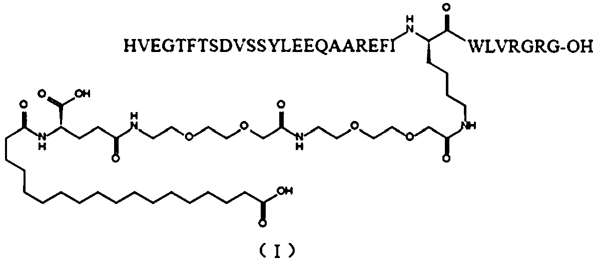
preferably, the protein or polypeptide drug and the delivery agent are in a mass ratio of 1:5-1:150;
preferably, the protein or polypeptide drug and the delivery agent are in a mass ratio of 1:10-1:50;
preferably, the protein or polypeptide drug is used in combination with the delivery synergist;
preferably, the combination use comprises simultaneously using the protein or polypeptide drug and the delivery synergist in the form of a pharmaceutical composition; or separately using the protein or polypeptide drug and the delivery synergist in sequence;
preferably, the combination use is to simultaneously use the protein or polypeptide drug and the delivery synergist in the form of a pharmaceutical composition;
preferably, the pharmaceutical composition further comprises a pharmaceutically acceptable carrier;
preferably, the pharmaceutically acceptable carrier comprises a lubricant, a filler, a disintegrant, a flavoring agent, a colorant, a binder, an forming agent, a buffer, a diluent, a solubilizer, a tonicity regulator, a surfactant, a preservative, an isotonic agent, a stabilizer, and/or a chelating agent;
preferably, the pharmaceutically acceptable carrier comprises a lubricant, a filler, and a disintegrant;
preferably, the lubricant comprises magnesium stearate, glyceryl tribehenate, and/or stearic acid;
preferably, the lubricant is selected from magnesium stearate;
preferably, the filler is selected from mannitol and/or microcrystalline cellulose;
preferably, the disintegrant is selected from croscarmellose sodium;
preferably, the pharmaceutical composition further comprises one or more active pharmaceutical ingredients for use in combination with the protein or polypeptide drug;
preferably, the active pharmaceutical ingredient is selected from an antidiabetic agent, an anti-obesity agent, an appetite-regulating agent, an antihypertensive agent, an agent for treating and/or preventing complications and conditions caused by or associated with diabetes, and an agent for treating and/or preventing complications and conditions caused by or associated with obesity;
preferably, a dosage form of the pharmaceutical composition comprises a tablet, a capsule, an emulsion, an aqueous suspension, a dispersant, or a powder;
preferably, the dosage form of the pharmaceutical composition is a tablet;
preferably, the tablet is prepared by a dry granulation process and/or a direct mixing process;
preferably, the tablet has a hardness of 30-90 N.

16. Use of the pharmaceutical composition according to any one of claims 11, 12, 14, and 15 in the manufacture of a medicament for preventing and/or treating a metabolism-related disease, a nerve-related disease, or a brain-related disease, wherein
preferably, the metabolism-related disease comprises a carbohydrate metabolism disorder and/or a lipid metabolism disorder;
preferably, the carbohydrate metabolism disorder comprises a disease related to abnormal blood glucose;
preferably, the disease related to abnormal blood glucose comprises diabetes, diabetic oculopathy, diabetic heart disease, diabetic nephropathy, diabetic neuropathy, necrosis of distal lower limbs, atherosclerosis, coronary heart disease, myocardial infarction, cerebral thrombosis, cerebral hemorrhage, cerebral embolism, osteoporosis, hyperlipidemia, hypertension, obesity, fatty liver, and/or cirrhosis;
preferably, the diabetes comprises hyperglycemia, type II diabetes, impaired glucose tolerance, type I diabetes, non-insulin-dependent diabetes, maturity-onset diabetes of the young (MODY), and/or gestational diabetes;
preferably, the lipid metabolism disorder comprises a disease related to abnormal body weight;
preferably, the disease related to abnormal body weight comprises anemia, gastroptosis, inflammatory bowel disease, dyspepsia, gastrointestinal ulcer, endocrine dyscrasia, osteoporosis, obesity, hypertension, hyperlipidemia, coronary heart disease, diabetes, fatty liver, joint deformity, pain, cancer, respiratory insufficiency, kidney diseases, hyperlipidemia, atherosclerosis, cirrhosis, angina pectoris, and/or myocardial infarction;
preferably, the nerve-related disease comprises a neurodegenerative disease;
preferably, the neurodegenerative disease comprises brain atrophy, cerebral ischemia, brain injury, epilepsy, Parkinson's disease, Alzheimer's disease, amyotrophic lateral sclerosis, multiple sclerosis, Huntington's disease, spinocerebellar ataxia, Pick's disease, bovine spongiform encephalopathy, and/or Creutzfeldt-Jakob disease.

17. A kit comprising the pharmaceutical composition according to any one of claims 11, 12, 14, and 15.
